(19) **Europäisches Patentamt**
**European Patent Office**
**Office européen des brevets**

(11) **EP 3 251 584 A1**

(12) **EUROPEAN PATENT APPLICATION**

(43) Date of publication:
06.12.2017 Bulletin 2017/49

(51) Int Cl.:
$A61B\ 3/113^{(2006.01)}$     $A61B\ 3/15^{(2006.01)}$

(21) Application number: 17167719.8

(22) Date of filing: 24.04.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(30) Priority: 31.05.2016 JP 2016109305

(71) Applicant: Fujitsu Limited
Kawasaki-shi, Kanagawa 211-8588 (JP)

(72) Inventors:
• **Yoshioka, Takahiro**
Kanagawa, 211-8588 (JP)
• **Nakashima, Satoshi**
Kanagawa, 211-8588 (JP)

(74) Representative: **Hoffmann Eitle**
**Patent- und Rechtsanwälte PartmbB**
**Arabellastraße 30**
**81925 München (DE)**

(54) **LINE-OF-SIGHT DETECTOR AND LINE-OF-SIGHT DETECTION METHOD**

(57) A line-of-sight detector (1) including: a storage unit (121) in which a plurality of eyeball features are registered in a mode of associating each of the plurality of eyeball features with a corneal radius; a feature amount calculator (103) configured to calculate a feature amount of an eyeball of a person who is gazing at a first gaze point of the gaze points in accordance with an image captured by the camera (201) that has a prescribed position relationship with the person; a feature determination unit (106) configured to determine an eyeball feature of the person in accordance with a feature amount of the eyeball that corresponds to the first gaze point in the plurality of eyeball features registered in the storage unit, and the calculated feature amount of the eyeball of the person; and a parameter calculator (108) configured to calculate a calibration parameter for a line of sight of the person in accordance with the feature amounts of the eyeball that are included in the determined eyeball feature.

F I G. 1

**Description**

FIELD

The embodiments discussed herein are related to a line-of-sight detector and a line-of-sight detection method.

BACKGROUND

A line-of-sight detection technology for detecting a line of sight of a person is applied to an input device that inputs information by using a line of sight, recording a line of sight during driving of a person who is driving a vehicle, or the like. As one of a method for detecting a line of sight, the pupil-corneal reflex method for detecting a line of sight of a person on the basis of a position of the pupil and the position of a corneal reflex in an image of the eyeball of a person is known.

In the line-of-sight detection technology, a process for calibrating the direction of a line of sight according to a feature of the eyeball (calibration) is performed in order to accurately detect a line of sight of a person. In calibration according to the pupil-corneal reflex method, a calibration parameter used to calibrate a line of sight is calculated on the basis of a line of sight that is detected according to the position of the pupil and the position of a corneal reflex in a state in which a person is made to be gazing at a prescribed gaze point, and the direction of the gaze point viewed from the person.

In performing calibration and calculating the calibration parameter, a person to be detected is made to gaze at a plurality of gaze points.

As the line-of-sight detection technology above, a technology is also known in which plural sets of parameters that correspond to a plurality of observers are stored and an optimum set of parameters is selected according to a feature of the eyeball of a current observer so as to calculate a line of sight (see, for example, Document 1). Even when a state of the eyeball of an observer to be detected changes due to a change in an observer, or the like, this type of line-of-sight detection technology enables a simple, quick, and accurate response to the change.

Further, as the line-of-sight detection technology above, a technology is known in which, even after an initial calibration is performed and a line of sight starts to be detected, calibration is dynamically repeated such that the accuracy of detection of a line of sight is enhanced (see, for example, Document 2).

Document 1: Japanese Laid-open Patent Publication No. 07-035966
Document 2: Japanese Laid-open Patent Publication No. 2000-010723

SUMMARY

It is an object in one aspect of the invention to reduce a burden on a person of a detection target at the time of calibration in detection of a line of sight.

According to an aspect of the embodiment, a line-of-sight detector includes: a memory in which a plurality of eyeball features are registered in a mode of associating each of the plurality of eyeball features with a corneal radius, each of the plurality of eyeball features including a set of feature amounts of an eyeball that are detected when respective gaze points that have been specified are gazed at from a point of view that has a prescribed position relationship with a camera; and a processor configured to perform a process for detecting a line of sight of a person that is photographed in an image captured by the camera, the processor being connected to the memory, wherein the process performed by the processor includes for a person who is gazing at a first gaze point of the gaze points, calculating a feature amount of an eyeball of the person in accordance with the image captured by the camera that has a prescribed position relationship with the person; determining an eyeball feature of the person in accordance with a feature amount of the eyeball that corresponds to the first gaze point in the plurality of eyeball features registered in the memory, and the calculated feature amount of the eyeball of the person; and calculating a calibration parameter for a line of sight of the person in accordance with the feature amounts of the eyeball that are included in the determined eyeball feature.

BRIEF DESCRIPTION OF DRAWINGS

FIG. 1 illustrates a functional configuration of a line-of-sight detector according to a first embodiment.
FIG. 2 illustrates an example of an eyeball feature database.
FIG. 3A is a diagram explaining a feature amount of the eyeball.
FIG. 3B is a diagram explaining an interpupillary distance.

FIG. 4 illustrates a functional configuration of a database generator.

FIG. 5 is a flowchart explaining an eyeball feature database generation process according to the first embodiment.

FIGS. 6A and 6B illustrate an application example of a line-of-sight detector.

FIGS. 7A and 7B are diagrams explaining a method for obtaining an image at the time of generating an eyeball feature database.

FIG. 8 is a flowchart explaining processing performed by the line-of-sight detector according to the first embodiment.

FIG. 9 is a flowchart explaining the content of a calibration parameter determination process according to the first embodiment.

FIG. 10 is a flowchart explaining the content of a line-of-sight detection process.

FIG. 11 is a diagram explaining a correlation between the position of the pupil and the position of a corneal reflex, and a corneal radius.

FIG. 12 is a diagram explaining a correlation between the position of the pupil and the position of a corneal reflex on an image and a corneal radius.

FIG. 13 illustrates another application example of a line-of-sight detector.

FIGS. 14A and 14B illustrate variations of a line-of-sight detector.

FIG. 15 is a flowchart explaining the content of a calibration parameter determination process according to a second embodiment.

FIG. 16 is a flowchart explaining the content of a process for correcting a feature amount of the eyeball.

FIG. 17 is a diagram explaining a relationship between the orientation of a face and a feature amount of the eyeball.

FIG. 18 is a diagram explaining a method for correcting a feature amount of the eyeball in a case in which a face does not face a camera.

FIG. 19 illustrates a functional configuration of a line-of-sight detector according to a third embodiment.

FIG. 20 illustrates an example of an eyeball feature database according to the third embodiment.

FIG. 21 is a diagram explaining a feature amount of the eyeball and an iris radius.

FIG. 22 is a flowchart explaining the content of a calibration parameter determination process according to the third embodiment.

FIG. 23 is a diagram explaining a correlation between an iris radius and the shape of the eyeball.

FIG. 24 is a diagram explaining an example of a method for determining an eyeball feature according to the third embodiment.

FIG. 25 is a flowchart explaining the content of a process for determining an eyeball feature in a calibration parameter determination process according to a fourth embodiment.

FIG. 26 illustrates examples of a plurality of records extracted from an eyeball feature database.

FIG. 27 is a diagram explaining a method for calculating a feature amount.

FIG. 28 illustrates a hardware configuration of a computer.

DESCRIPTION OF EMBODIMENTS

Preferred embodiments of the present invention will be explained with reference to accompanying drawings.

In performing calibration and calculating a calibration parameter for a line of sight, a process for specifying a gaze point, making a person gaze at the gaze point, and obtaining an image in a state in which the person is gazing at the gaze point may be performed multiple times, while changing the gaze point. In this case, a person who is a target for detection of a line of sight gazes at a plurality of gaze points (for example, four to nine gaze points) in one calibration. Gazing at a plurality of gaze points, as described above, imposes a burden on a person of a detection target. Embodiments of calibration that enables a burden on the person of the detection target to be reduced are described below.

<First Embodiment>

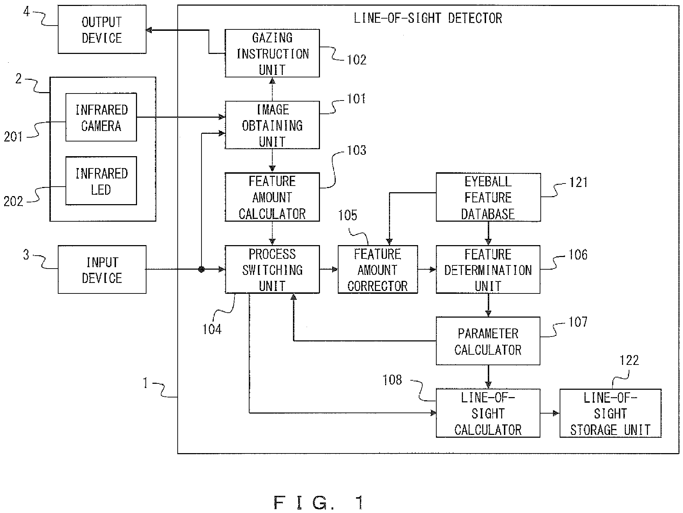

FIG. 1 illustrates a functional configuration of a line-of-sight detector according to a first embodiment.

As illustrated in FIG. 1, a line-of-sight detector 1 according to this embodiment includes an image obtaining unit 101, a gazing instruction unit 102, a feature amount calculator 103, a process switching unit 104, a feature amount corrector 105, a feature determination unit 106, a parameter calculator 107, and a line-of-sight calculator 108. The line-of-sight detector 1 further includes an eyeball feature database 121 and a line-of-sight storage unit 122. The line-of-sight detector 1 according to this embodiment detects a line of sight of a person who is a target for detection of a line of sight (hereinafter referred to as a target person) according to the pupil-corneal reflex method.

The image obtaining unit 101 obtains an infrared image (hereinafter simply referred to as an image) that is captured by using an infrared camera 201. The infrared camera 201 is an imaging device used to capture an image including the eyeball of a target person who observes a prescribed space area or a display surface of a prescribed display device. The infrared camera 201 is used in combination with a light source that emits infrared light, such as an infrared light emitting diode (LED) 202. In a pair of the infrared camera 201 and the infrared LED 202 (hereinafter referred to as a line-of-sight sensor 2), the infrared LED 202 is installed near the infrared camera 201 in a mode of emitting infrared light toward a space including an imaging range of the infrared camera 201. The infrared camera 201 captures an image including the eyeball of the target person that is irradiated with the infrared light emitted by the infrared LED 202, and transmits the image to the image obtaining unit 101.

In a case in which the line-of-sight detector 1 performs a process for calibrating a line of sight of the target person (calibration), the image obtaining unit 101 makes the gazing instruction unit 102 perform a process for making the target person gaze at a gaze point. The process herein for calibrating the line of sight refers to a process for determining a calibration parameter that is used in a process for detecting the line of sight of the target person.

The image obtaining unit 101 determines whether the process for making the target person gaze at the gaze point will be performed, on the basis of information that is input from an input device 3. The input device 3 is a button switch, a touch panel device, or the like, and the input device 3 is used to input, for example, an instruction to make the line-of-sight detector 1 start a line-of-sight detection process, an instruction to make the line-of-sight detector 1 perform calibration of a line of sight, or other instructions. In a case in which the information that is input from the input device 3 is an instruction to perform processing including calibration of a line of sight, the image obtaining unit 101 makes the gazing instruction unit 102 perform a process for making the target person gaze at the gaze point.

The gazing instruction unit 102 performs a process for reporting, to the target person, a position that the target person is made to gaze at (a gaze point), by using an output device 4. As an example, in a case in which the output device 4 is a display device, the gazing instruction unit 102 displays an image indicating the gaze point on a display surface of the display device. In a case in which the output device 4 is a speaker, the gazing instruction unit 102 outputs a prepared sound signal to the speaker.

The feature amount calculator 103 calculates a feature amount of the eyeball of the target person photographed in an image obtained from the infrared camera 201 on the basis of the image. The feature amount calculator 103 extracts, from the image, the center of a corneal reflex and the center of the pupil of each of a right eye and a left eye, and calculates a position relationship between them as a feature amount of the eyeball. It is assumed, for example, that a position relationship between the center of the corneal reflex and the center of the pupil is two-dimensional coordinates representing a center position of the pupil viewed from a center position of the corneal reflex in the image.

The process switching unit 104 switches a process for calibrating a line of sight and a process for detecting a line of sight in accordance with the information that is input from the input device 3 and information that is input from the parameter calculator 107. In a case in which the information that is input from the input device 3 includes information indicating an instruction to perform the process for calibrating the line of sight, and a calibration parameter after inputting the information has not yet been determined, the process switching unit 104 switches a process performed by the line-of-sight detector 1 to calibration (the process for calibrating the line of sight). In a case in which the information that is input from the input device 3 does not include the information indicating the instruction to perform the process for calibrating the line of sight, or in a case in which the calibration parameter has already been determined, the process switching unit 104 switches the process performed by the line-of-sight detector 1 to a process for calculating a line of sight.

The feature amount corrector 105 calculates a distance between the pupils of both eyes on the basis of the positions of the pupils that are extracted by the feature amount calculator 103, and corrects the feature amount of the eyeball as needed. In a case in which a calculated interpupillary distance is different from a preset interpupillary distance, the feature amount corrector 105 corrects the feature amount of the eyeball in accordance with a ratio of the calculated interpupillary distance and the preset interpupillary distance. The preset interpupillary distance has been registered in the eyeball feature database 121.

The feature determination unit 106 determines an eyeball feature of the target person on the basis of the feature amount of the eyeball that is calculated by the feature amount calculator 103 (a corrected feature amount of the eyeball in a case in which correction is performed by the feature amount corrector 105) and the eyeball feature database 121. In the eyeball feature database 121, feature amounts of the eyeball at the time when each of a plurality of persons that have different eyeball features from each other gazes at respective gaze points have been registered. The feature determination unit 106 determines the eyeball feature of the target person on the basis of a feature amount of the eyeball that is calculated from an image at the time when the target person is gazing at any one of the gaze points (or the feature amount of the eyeball corrected by the feature amount corrector 105) and the eyeball feature database 121. Namely, the feature determination unit 106 determines a feature amount of the eyeball at the time when the target person is gazing at each of the gaze points on the basis of a feature amount of the eyeball at the time when the target person is gazing at any one of the gaze points and the eyeball feature database 121.

The parameter calculator 107 calculates a calibration parameter for a line of sight of the target person on the basis of the eyeball feature determined by the feature determination unit 106. After calculating the calibration parameter, the parameter calculator 107 transmits the calculated calibration parameter to the line-of-sight calculator 108, and reports to the process switching unit 104 that the calibration parameter has been calculated.

The line-of-sight calculator 108 calculates the line of sight of the target person on the basis of the feature amount of the eyeball calculated by the feature amount calculator 103 and the calibration parameter. The line-of-sight calculator 108 stores the calculated line of sight of the target person in the line-of-sight storage unit 122 in time series.

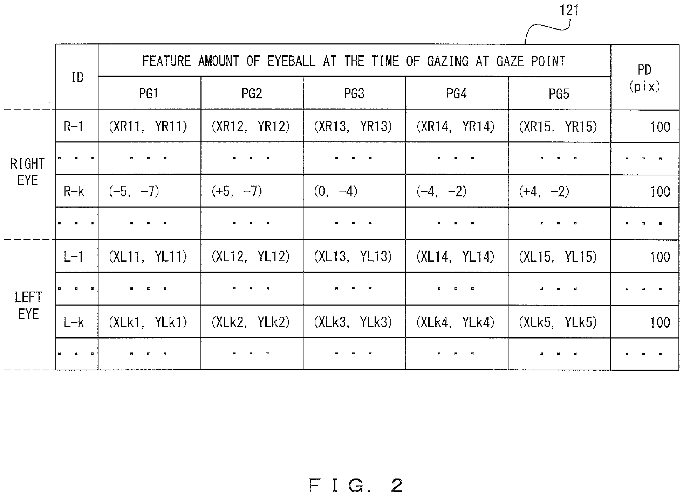

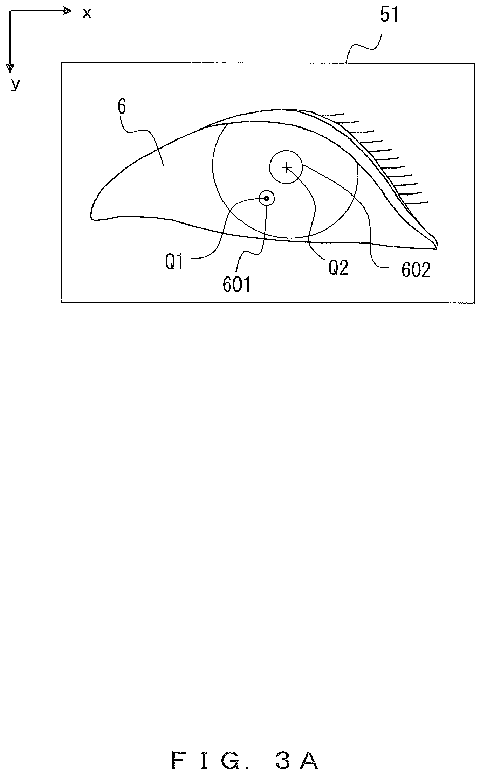

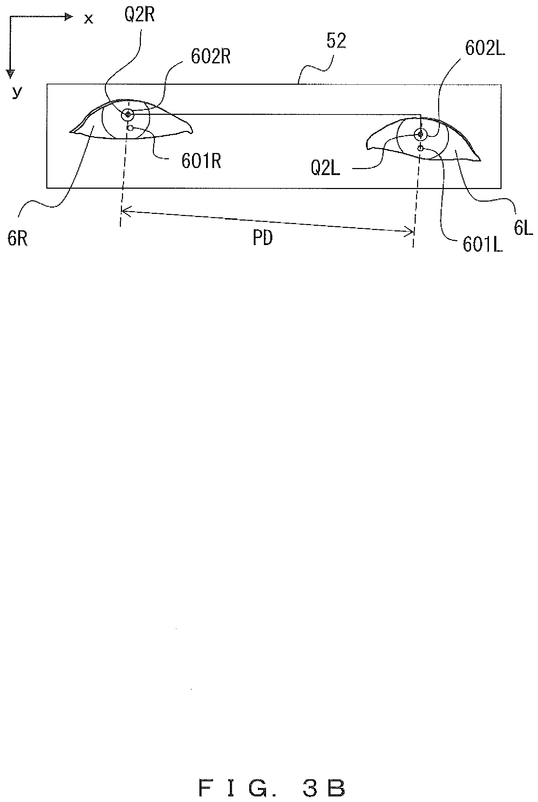

FIG. 2 illustrates an example of an eyeball feature database. FIG. 3A is a diagram explaining a feature amount of the eyeball. FIG. 3B is a diagram explaining an interpupillary distance.

As illustrated in FIG. 2, the eyeball feature database 121 includes an identifier (ID), a feature amount of the eyeball (PG1 to PG5) at the time of gazing at a gaze point, and an interpupillary distance (PD).

The identifier (ID) is a value for identifying a record including an eyeball feature, the record being registered in the eyeball feature database 121. In the eyeball feature database 121 of FIG. 2, eyeball features for one person are classified into eyeball features of a right eye and eyeball features of a left eye, and individual identifiers are assigned to the respective eyeball features.

The feature amount of the eyeball at the time of gazing at a gaze point is two-dimensional coordinates representing a relative position of the center of the pupil viewed from the center of a corneal reflex in an image captured at the time of gazing at the gaze point. In the eyeball feature database 121 of FIG. 2, respective feature amounts of the eyeball for five gaze points PG1 to PG5 have been registered as an eyeball feature for one identifier (record).

The interpupillary distance (PD) is a distance between the pupil of the right eye and the pupil of the left eye in an image used to generate the eyeball feature database 121. An image of a person that is captured in a state in which a distance from the infrared camera 201 to the person is a prescribed distance (for example, 600 mm) is used to generate the eyeball feature database 121. Stated another way, the interpupillary distance in the eyeball feature database 121 is an interpupillary distance at the time when the distance between the infrared camera 201 to the person is the prescribed distance.

As described above, each of a plurality of records registered in the eyeball feature database 121 includes feature amounts of the eyeball at the time when a person gazes at respective five gaze points PG1 to PG5, and an interpupillary distance.

An image obtained by the line-of-sight detector 1 is an image including the eyeball of a target person that is captured by the infrared camera 201 in a state in which a face of the target person is irradiated with infrared light. Accordingly, a corneal reflex 601 of infrared light is photographed in a first partial region 51 including the eyeball 6 in the obtained image, as illustrated in FIG. 3A.

The line-of-sight detector 1 that detects a line of sight according to the pupil-corneal reflex method detects a line of sight, for example, on the basis of a relative position of the center Q2 of the pupil 602 viewed from a reference point Q1 when the center Q1 of the corneal reflex 601 is used as a reference point. At this time, the line-of-sight detector 1 performs calibration in order to reduce an error between a line of sight that is calculated on the basis of the first partial region 51 in the image and an actual line of sight of the target person and to accurately detect a line of sight.

The line-of-sight detector 1 according to this embodiment obtains an image in which a target person is gazing at any one point of a prescribed plurality of gaze points, and performs calibration on the basis of feature amounts of the eyeballs in the image and the eyeball feature database 121. It is assumed that the gaze point that the target person is made to gaze at is any one point of a plurality of gaze points that are set when the eyeball feature database 121 is generated. In a case in which calibration is performed by using the eyeball feature database 121 of FIG. 2, the line-of-sight detector 1 obtains an image that the target person is gazing at any one of the five gaze points PG1 to PG5, and performs calibration.

The line-of-sight detector 1 obtains an image captured in a state in which the target person is gazing at a specified point (a gaze point), and calculates a feature amount of the right eye and a feature amount of the left eye in the obtained image. Then, the line-of-sight detector 1 corrects the feature amount of the right eye and the feature amount of the left eye in the image, as needed, on the basis of an interpupillary distance in the image and an interpupillary distance in the eyeball feature database 121. As illustrated in FIG. 3B, a right eye 6R and a left eye 6L of the target person are photographed in a second partial region 52 in the image obtained from the infrared camera 201. An interpupillary distance PD is a distance (a pixel difference) between the center Q2R of the pupil 602R of the right eye 6R and the center Q2L of the pupil 602L of the left eye 6L on the image. Assume that a pixel including the center Q2R of the pupil 602R of the right eye 6R in the image is represented by the coordinates (PRx, PRy), and that a pixel including the center Q2L of the pupil 602L of the left eye 6L is represented by the coordinates (PLx, PLy). The interpupillary distance PD is calculated according to Expression (1) below.

$$PD = \sqrt{\left(PRx - PLx\right)^2 + \left(PRy - PLy\right)^2} \qquad (1)$$

As an example, in a case in which the pixel including the center Q2R of the pupil 602R of the right eye 6R in the image is a pixel represented by the coordinates (173, 124), and the pixel including the center Q2L of the pupil 602L of the left eye 6L is a pixel represented by the coordinates (275, 131), the interpupillary distance PD is 102.2 (pix). In this case, an interpupillary distance calculated from the image is different from an interpupillary distance in the eyeball feature database 121. Therefore, the line-of-sight detector 1 (the feature amount corrector 105) corrects the calculated feature amounts of the eyeballs in accordance with a ratio of the calculated interpupillary distance and the interpupillary distance in the database. As an example, in a case in which a feature amount of the right eye calculated from the image is (0, -2), the feature amount corrector 105 corrects the feature amount to (0×100/102.2, -2×100/102.2) ≈ (0, -1.95) in accordance with a ratio of interpupillary distances.

In a case in which the feature amount of the eyeball is corrected, the line-of-sight detector 1 (the feature determination unit 106) refers to a feature amount of the eyeball of a gaze point that the target person is made to gaze at in the eyeball feature database 121, and extracts a record including a feature amount that matches the corrected feature amount. The extracted record includes feature amounts of the eyeball at the time of gazing at the five gaze points PG1 to PG5. Accordingly, the feature determination unit 106 respectively determines five feature amounts of the eyeball that are included in the extracted record to be feature amounts of the eyeball at the time when the target person gazes at the five gaze points PG1 to PG5. The feature determination unit 106 determines feature amounts of the eyeball for each of the right eye and the left eye at the time when the target person gazes at the five gaze points PG1 to PG5. Then, the line-of-sight detector 1 (the parameter calculator 107) calculates a calibration parameter in accordance with five feature amounts of the eyeball for the right eye and five feature amounts of the eyeball for the left eye that have been determined.

As described above, the line-of-sight detector 1 according to this embodiment determines feature amounts of the eyeballs at the time when a target person is gazing at respective gaze points in accordance with an image captured when the target person is gazing at one gaze point and the eyeball feature database 121, and calculates a calibration parameter. Namely, in the line-of-sight detector 1 according to this embodiment, one gaze point may be gazed at by a target person in one calibration. Thus, according to this embodiment, a burden of gazing at a gaze point that is imposed on a target person at the time of calibration can be reduced.

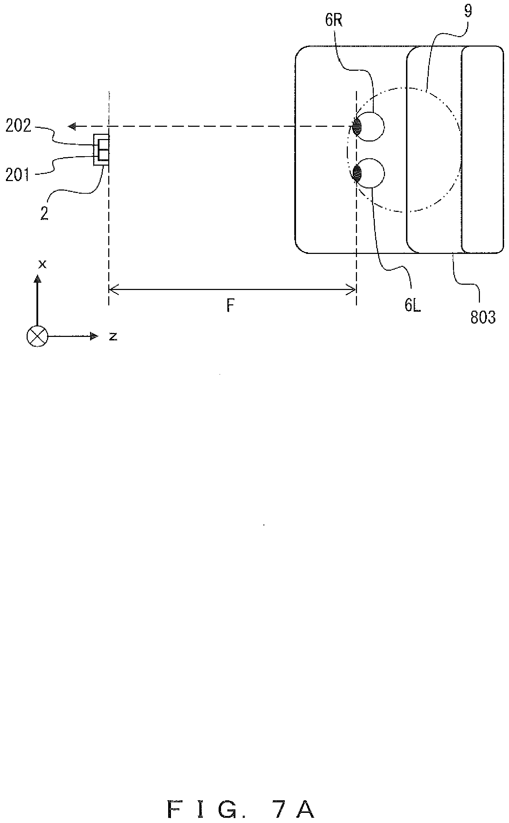

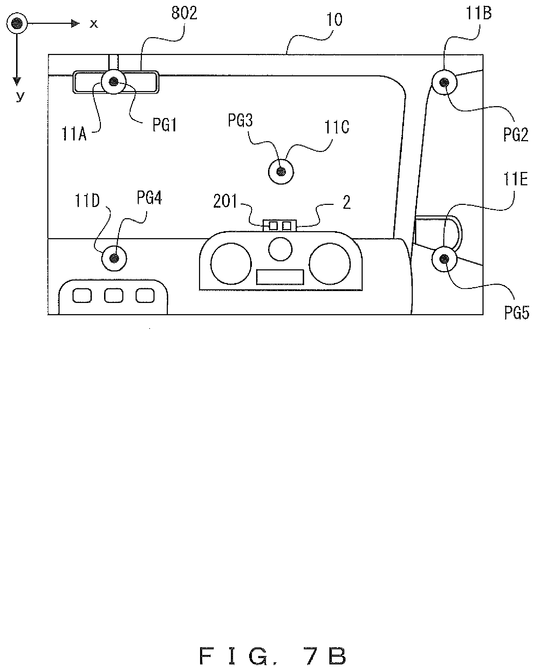

The eyeball feature database 121 above is generated, for example, by a database generator 7 illustrated in FIG. 7.

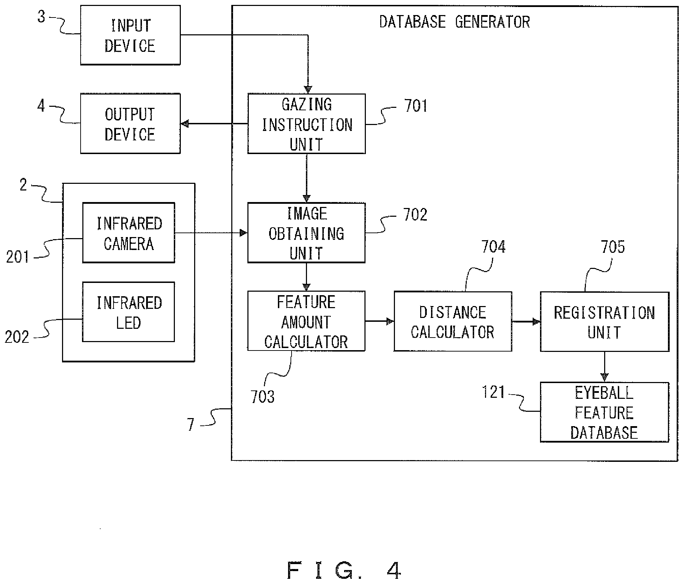

FIG. 4 illustrates a functional configuration of a database generator.

As illustrated in FIG. 4, the database generator 7 includes a gazing instruction unit 701, an image obtaining unit 702, a feature amount calculator 703, a distance calculator 704, a registration unit 705, and an eyeball feature database 121.

When an instruction to start a process for registering an eyeball feature is input from the input device 3, the gazing instruction unit 701 generates information indicating a gaze point to be gazed at by a target person, and transmits the generated information to the output device 4. After transmitting the information indicating the gaze point to the output device 4, the gazing instruction unit 701 makes the image obtaining unit 702 obtain an image of the target person.

The image obtaining unit 702 obtains an image including the eyeball of the target person that have been captured by the infrared camera 201 of the line-of-sight sensor 2. As the line-of-sight sensor 2 (the infrared camera 201 and the infrared LED 202), a line-of-sight sensor is used that can capture an image on the same condition as the condition of the line-of-sight sensor 2 that is used in combination with the line-of-sight detector 1.

The feature amount calculator 703 extracts the pupil and a corneal reflex from the image obtained from the infrared camera 201, and calculates a feature amount of the eyeball of the target person photographed in the image. The feature amount calculator 703 of the database generator 7 calculates, as the feature amount of the eyeball, a value indicating a position relationship between the center of the corneal reflex and the center of the pupil for each of the right eye and the left eye, similarly to the feature amount calculator 103 of the line-of-sight detector 1. The position relationship between the center of the corneal reflex and the center of the pupil is assumed to be, for example, two-dimensional coordinates representing a relative position of the center of the pupil viewed from the center of the corneal reflex.

The distance calculator 704 calculates an interpupillary distance (a pixel difference) PD between the center of the pupil of the right eye and the center of the pupil of the left eye in the image.

The registration unit 705 registers a pair of the feature amount of the eyeball that is calculated by the feature amount calculator 703 and the interpupillary distance that is calculated by the distance calculator 704 in the eyeball feature database 121. The registration unit 705 assigns an identifier for the right eye to a pair of a feature amount of the right eye and the interpupillary distance, and registers them as one record in the eyeball feature database 121. The registration unit 705 also assigns an identifier for the left eye to a pair of a feature amount of the left eye and the interpupillary distance, and registers them as one record in the eyeball feature database 121.

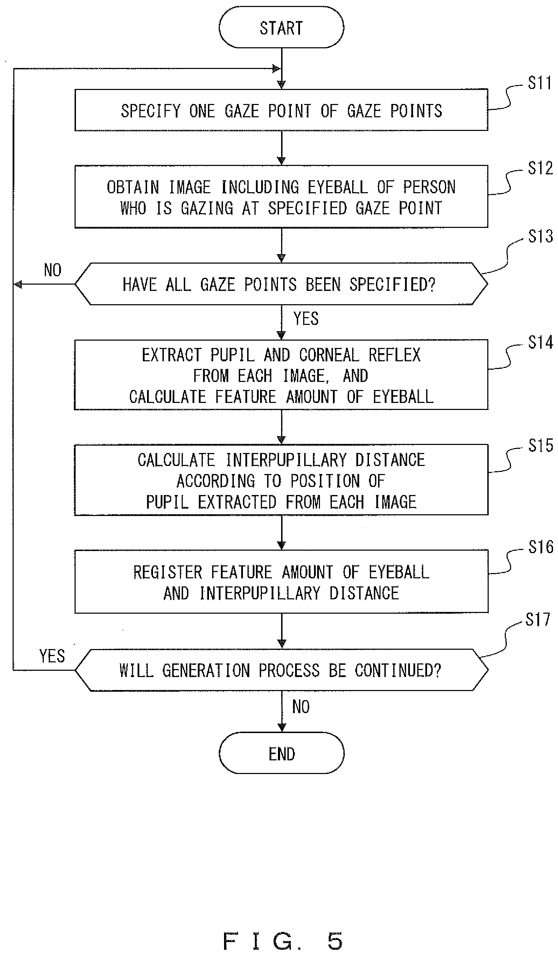

When an operator of the database generator 7 or the target person operates the input device 3, and inputs an instruction to start a process for registering an eyeball feature, the database generator 7 performs the processing illustrated in FIG. 5.

FIG. 5 is a flowchart explaining an eyeball feature database generation process according to the first embodiment.

The database generator 7 first specifies one gaze point of a plurality of gaze points (step S11), and obtains an image including the eyeball of a person who is gazing at the specified gaze point (step S12). Then, the database generator 7 determines whether all of the plurality of gaze points have been specified (step S13). When there are any gaze points that have not yet been specified (step S13; NO), the database generator 7 repeats the processes of steps S11 and S12. The process of step S11 is performed by the gazing instruction unit 701. The process of step S12 is performed by the image obtaining unit 702 in cooperation with the infrared camera 201. The determination in step S13 is performed by the gazing instruction unit 701 and the image obtaining unit 702.

In step S11, the gazing instruction unit 701 specifies one gaze point of a preset plurality of gaze points according to a prescribed specification rule. It is assumed, for example, that the specification rule is a rule that the first gaze point in the preset specification order of gaze points that have not yet been specified in a process for one person is specified.

After specifying the gaze point, the gazing instruction unit 701 reports the specified gaze point to the person by using the output device 4, and makes the person gaze at the specified gaze point. As an example, in a case in which the output device 4 is a speaker, the gazing instruction unit 701 outputs, to the output device 4, a sound signal including sound for making the person gaze at the specified gaze point. In a case in which the output device 4 is a display device, the gazing instruction unit 701 outputs, to the output device 4, an image signal including an image representing the gaze point. After making the person gaze at the gaze point by using the output device 4, the gazing instruction unit 701 makes the image obtaining unit 702 obtain an image including the eyeball of the person who is gazing at the gaze point.

In step S12, the image obtaining unit 702 makes the infrared camera 201 of the line-of-sight sensor 2 capture an image, and obtains the image captured by the infrared camera 201.

The gazing instruction unit 701 and the image obtaining unit 702 repeats the processing above until the gazing instruction unit 701 and the image obtaining unit 702 specify all of the preset plurality of gaze points, and obtain a plurality of images in which one person is gazing at the respective plurality of gaze points. In a case in which the determination in step S13 is performed by the gazing instruction unit 701, the image obtaining unit 702 reports to the gazing instruction unit 701 that an image has been obtained, every time the image is obtained. Upon receipt of the report from the image obtaining unit 702, the gazing instruction unit 701 performs the determination in step S13. When there are any gaze points that have not yet been specified (step S13; NO), the gazing instruction unit 701 specifies the next gaze point, makes the target person gaze at the gaze point, and makes the image obtaining unit 702 obtain an image. When all of the gaze points have already been specified (step S13; YES), the database generator 7 extracts the pupil and a corneal reflex from each of the plurality of images, and calculates a feature amount of the eyeball (step S14).

In a case in which the determination in step S13 is performed by the image obtaining unit 702, the image obtaining unit 702 determines whether all of the images in which one person is gazing at all respective gaze points have been obtained, as the determination in step S13. When there are any gaze points for which images have not yet been obtained (step S13; NO), the image obtaining unit 702 makes the gazing instruction unit 701 specify the next gaze point. When all of the gaze points have been gazed at, and all of the images have been obtained (step S13; YES), the database generator 7 extracts the pupil and a corneal reflex from each of the images, and calculates the feature amount of the eyeball (step S14).

The process of step S14 is performed by the feature amount calculator 703. The feature amount calculator 703 extracts the pupil and a corneal reflex of the right eye and the pupil and a corneal reflex of the left eye from the obtained image in accordance with a method for extracting the pupil and a corneal reflex in a known pupil-corneal reflex method (see FIG. 3A). Then, the feature amount calculator 703 calculates a feature amount of the eyeball of the right eye (a feature amount of the right eye) at the time of gazing at each of the gaze points, in accordance with a position relationship between the center of the corneal reflex and the center of the pupil of the right eye. The feature amount extraction unit 703 also calculates a feature amount of the eyeball of the left eye (a feature amount of the left eye) at the time of gazing at each of the gaze points, in accordance with a position relationship between the center of the corneal reflex and the center of the pupil of the left eye. The feature amount calculator 703 transmits, to the distance calculator 704, the feature amount of the right eye and the feature amount of the left eye that have been extracted from each of the images in association with a gaze point that the person is gazing at the time of photographing each of the images.

When the process of step S14 is finished, the database generator 7 calculates an interpupillary distance on the basis of the positions of the pupils extracted from each of the images (step S15). The process of step S15 is performed by the distance calculator 704. As an example, the distance calculator 704 first calculates an interpupillary distance (the number of pixels) PD between the center of the pupil of the right eye and the center of the pupil of the left eye in each of the images for different gaze points. Then, the distance calculator 704 calculates an average value of the calculated interpupillary distances, and specifies the average value of the interpupillary distances to be an interpupillary distance of a person photographed in the images. The distance calculator 704 transmits, to the registration unit 705, the feature amounts of the right eye and the left eye that have been received from the feature amount calculator 703 and the interpupillary distances calculated by the registration unit 705.

When the process of step S15 is finished, the database generator 7 performs a process for registering the feature amount of the right eye and the feature amount of the left eye that have been calculated from each of the images, and the interpupillary distance in the eyeball feature database 121 by using the registration unit 705 (step S16). The registration unit 705 assigns an identifier for the right eye to a set of the feature amount of the right eye at the time of gazing at each of the gaze points and the interpupillary distance, and registers them as one record in the eyeball feature database 121. At this time, each of the feature amounts of the right eye is registered in a field of a gaze point that is associated with each of the feature amounts of the right eye from among a plurality of gaze points in the eyeball feature database 121. The registration unit 705 also assigns an identifier for the left eye to a set of the feature amount of the left eye at the time of gazing at each of the gaze points and the interpupillary distance, and registers them as one record in the eyeball feature database 121. At this time, each of the feature amounts of the left eye is registered in a field of a gaze point that is associated with each of the feature amounts of the left eye from among a plurality of gaze points in the eyeball feature database 121.

When the process of step S16 is finished, the database generator 7 determines whether the eyeball feature database generation process will be continued (step S17). In the determination in step S17, the database generator 7 awaits, for example, an input of information indicating whether the generation process will be continued. As an example, when an operator operates the input device 3, and inputs information indicating that the generation process will be continued, namely, when the generation process is continued (step S17; YES), the database generator 7 performs the processes of steps S11 to S16. When the operator operates the input device 3, and inputs information indicating that the generation process will be finished, namely, when the generation process is not continued (step S17; NO), the database generator 7 finishes the generation process.

The eyeball feature database 121 generated according to the procedure above is transferred to the line-of-sight detector 1, for example, via a portable recording medium, such as a memory card mounted with a flash memory or an optical disk, or a transmission cable, and is stored in a storage unit of the line-of-sight detector 1.

In generating the eyeball feature database 121, feature amounts of the eyeball and an interpupillary distance at the time when each of a plurality of persons having different corneal radiuses (different sizes of the eyeball) from each other gazes at a plurality of gaze points are calculated and registered, as described above. At this time, it is preferable, for example, that corneal radiuses be set at 0.1 mm intervals within a range of 6.0 mm to 9.0 mm, and that feature amounts of the eyeball for each of the corneal radiuses be registered in the eyeball feature database 121. The corneal radius can be measured by using a dedicated measurement device such as a keratometer. The feature amounts of the eyeball that are registered in the eyeball feature database 121 maybe calculated by using, for example, an eyeball model reproduced on a computer, without calculating the feature amounts from images in which the eyeball of a person is photographed.

In addition, prepared information relating to an eyeball feature that includes a set of feature amounts of the eyeball at the time of gazing at respective gaze points is not limited to the eyeball feature database 121 of FIG. 2, and the information may be registered (stored) in a storage unit in another mode in which the feature amounts can be discriminated from each other.

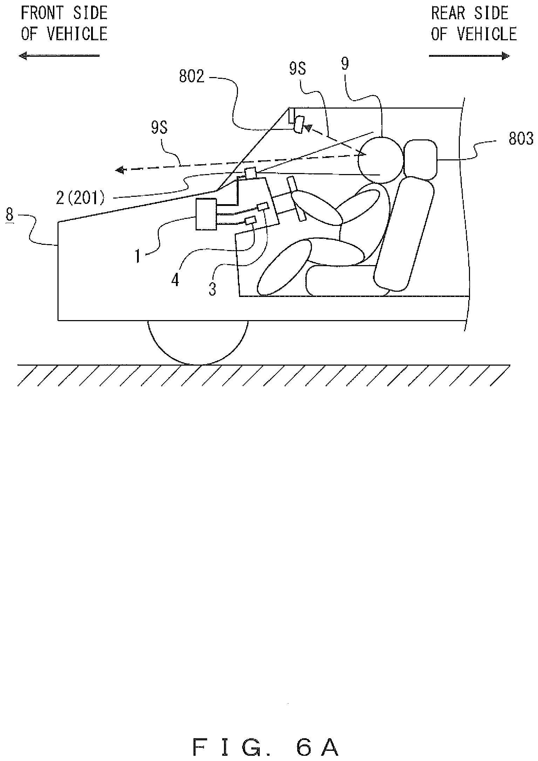

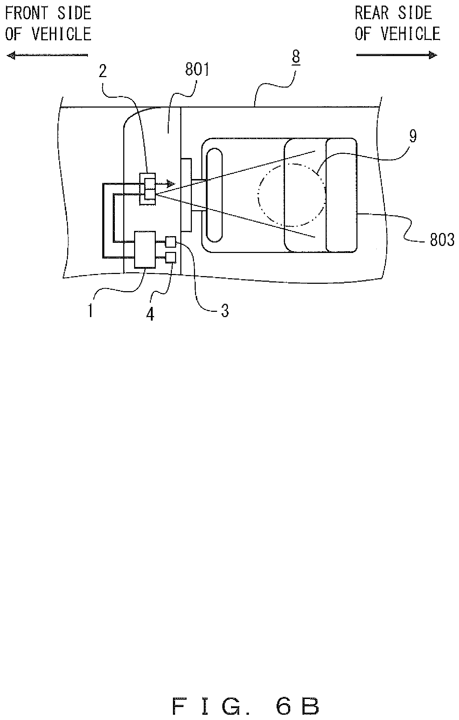

FIGS. 6A and 6B illustrate an application example of a line-of-sight detector. FIG. 6A schematically illustrates the periphery of a driver at the time of viewing a vehicle 8 mounted with the line-of-sight detector 1 from a left-hand side of the vehicle. FIG. 6B schematically illustrates the periphery of a driver at the time of viewing the vehicle 8 of FIG. 6A from above.

The line-of-sight detector 1 according to this embodiment can be applied, for example, to a line-of-sight detection system that detects a line of sight 9S of a target person (a driver) 9 who drives the vehicle 8, as illustrated in FIGS. 6A and 6B. This type of line-of-sight detection system includes a line-of-sight detector 1, a line-of-sight sensor 2, an input device 3, and an output device such as a speaker 4.

The line-of-sight sensor 2 is installed, for example, on a dashboard 801 of the vehicle 8 so as to face the target person 9. The line-of-sight detector 1, the input device 3, and the speaker 4 are installed, for example, in a center console of the vehicle 8. The line-of-sight detector 1 may be incorporated, for example, into a drive recorder (an event data recorder) that records a driving state of the vehicle 8 or information around the vehicle 8.

The line of sight 9S of the driver (the target person 9) at the time of driving the vehicle 8 is directed toward not-illustrated instruments (an instrument panel), a room mirror 802 in a vehicle cabin, a not-illustrated door mirror, and the like, in addition to a front side of the vehicle. The line-of-sight detection system detects, for example, the line of sight 9S of the target person 9 during driving, and stores the history of the line of sight. The history of the line of sight stored by the line-of-sight detection system is used, for example, to determine whether the target person 9 is appropriately moving a line of sight in order to drive safely.

The eyeball feature database 121 used in the line-of-sight detector 1 that detects the line of sight 9S of the driver (the target person 9) who drives the vehicle 8 is generated, for example, by obtaining an image of a person seated on a driver's seat 803 of the vehicle 8.

FIGS. 7A and 7B are diagrams explaining a method for obtaining an image at the time of generating an eyeball feature database. FIG. 7A illustrates a distance from an infrared camera 201 to eyeballs 6R and 6L of a person 9 at the time of obtaining an image. FIG. 7B illustrates an example of setting a plurality of gaze points.

In a case in which the line-of-sight detector 1 is used to detect the line of sight 9S of the target person 9 who is driving the vehicle 8, the eyeball feature database 121 is generated by obtaining an image at the time when a person seated on the driver's seat 803 in the same type of vehicle as that of the vehicle 8 is gazing at a gaze point. At this time, the line-of-sight sensor 2 is installed in almost the same position as a position in which the line-of-sight sensor 2 is installed in the line-of-sight detection system, namely, a position on the dashboard 801 that faces the person 9 seated on the driver' s seat 803. In addition, the position of the driver's seat 803 is adjusted in such a way that a distance F from the infrared camera 201 of the line-of-sight sensor 2 to the eyeballs 6R and 6L of the person 9 seated on the driver' s seat 803 is a prescribed distance (for example, 600 mm), as illustrated in FIG. 7A.

In this state, when the line-of-sight sensor 2, the input device 3, the speaker 4, and the like are connected to the database generator 7, and an instruction to start processing is input from the input device 3, the database generator 7 generates the eyeball feature database 121 according to the flowchart of FIG. 5. At this time, the database generator 7 makes the person 9 sequentially gaze at a plurality of gaze points that are set, for example, in positions on a front side of the vehicle that are located ahead of the eyeballs 6R and 6L of the person 9, and obtains images of the person 9 at the time of gazing at the respective gaze points. It is assumed, for example, that the plurality of gaze points are five points including four corners, PG1, PG2, PG4, and PG5, in a field of view 10 at the time when the person 9 is looking at the front of the vehicle, and the center PG3 of the field of view 10. The five gaze points PG1 to PG5 are provided, for example, by respectively attaching sticker-like markers 11A to 11E to points in a vehicle cabin.

As an example, the database generator 7 first makes the person 9 gaze at an upper-left gaze point PG1, and obtains an image captured by the infrared camera 201 in this state. Then, the database generator 7 makes the person 9 gaze at an upper-right gaze point PG2, and obtains an image captured by the infrared camera 201 in this state. After that, the database generator 7 sequentially obtains an image captured at the time when the person is gazing at the center PG3 of the field of view 10, an image captured at the time when the person 9 is gazing at a lower-left gaze point PG4, and an image captured at the time when the person 9 is gazing at a lower-right gaze point PG5. The database generator 7 then calculates feature amounts of the right eye and the left eye and also calculates an interpupillary distance from each of the obtained five images, and the database generator 7 registers them in the eyeball feature database 121.

The gaze points PG1 to PG5 illustrated in FIG. 7B are examples, and the positions or shapes of the gaze points PG1 to PG5, a method for presenting gaze points, and the number of gaze points can be changed appropriately.

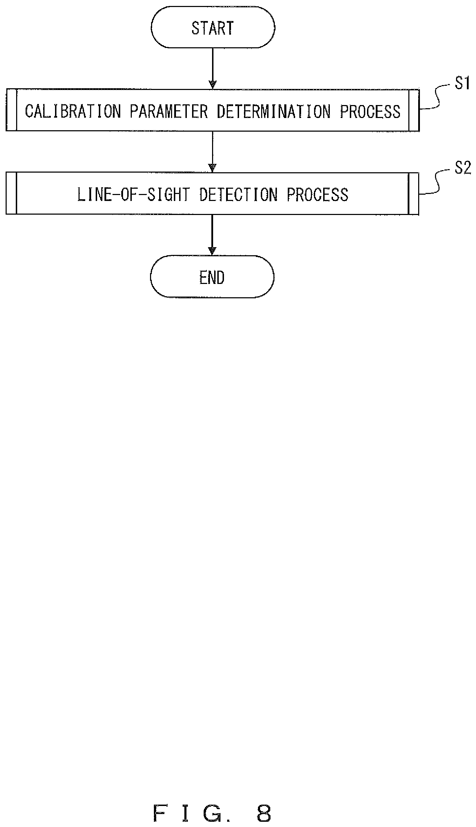

The line-of-sight detector 1 including the eyeball feature database 121 generated according to the method above performs the processing illustrated in FIG. 8, for example, when the target person (the driver) 9 operates the input device 3, and inputs an instruction to start a process for detecting a line of sight.

FIG. 8 is a flowchart explaining processing performed by the line-of-sight detector according to the first embodiment.

As illustrated in FIG. 8, when the line-of-sight detector 1 start an operation, the line-of-sight detector 1 first performs a calibration parameter determination process (step S1). In step S1, the line-of-sight detector 1 specifies any one gaze point (a first gaze point) of a plurality of gaze points that were set at the time of generating the eyeball feature database 121, and obtains an image at the time when the target person is gazing at the specified gaze point. Then, the line-of-sight detector 1 determines an eyeball feature of the target person on the basis of a center position of the pupil and a center position of a corneal reflex in the image of the target person who is gazing at the specified gaze point, and the eyeball feature database 121. The determined eyeball feature includes a plurality of feature amounts of the eyeball that correspond to feature amounts of the eyeball at the time when the target person gazes at the respective gaze points. Accordingly, after determining the eyeball feature, the line-of-sight detector 1 calculates a calibration parameter according to a known calculation method for making a target person gaze at a plurality of gaze points and calculating a calibration parameter in the pupil-corneal reflex method.

The process of step S1 is performed by the image obtaining unit 101, the gazing instruction unit 102, the feature amount calculator 103, the feature amount corrector 105, the feature determination unit 106, and the parameter calculator 107.

After determining the calibration parameter in step S1, the line-of-sight detector 1 performs a line-of-sight detection process (step S2). In step S2, the line-of-sight detector 1 detects (calculates) a line of sight of the target person on the basis of a center position of the pupil and a center position of a corneal reflex in the image of the target person, and the calibration parameter determined in step S1. In step S2, the line-of-sight detector 1 calculates the direction, position, and the like of the line of sight of the target person in accordance with a known method for calculating a line of sight in the pupil-corneal reflex method.

The process of step S2 is performed by the image obtaining unit 101, the feature amount calculator 103, and the line-of-sight calculator 108.

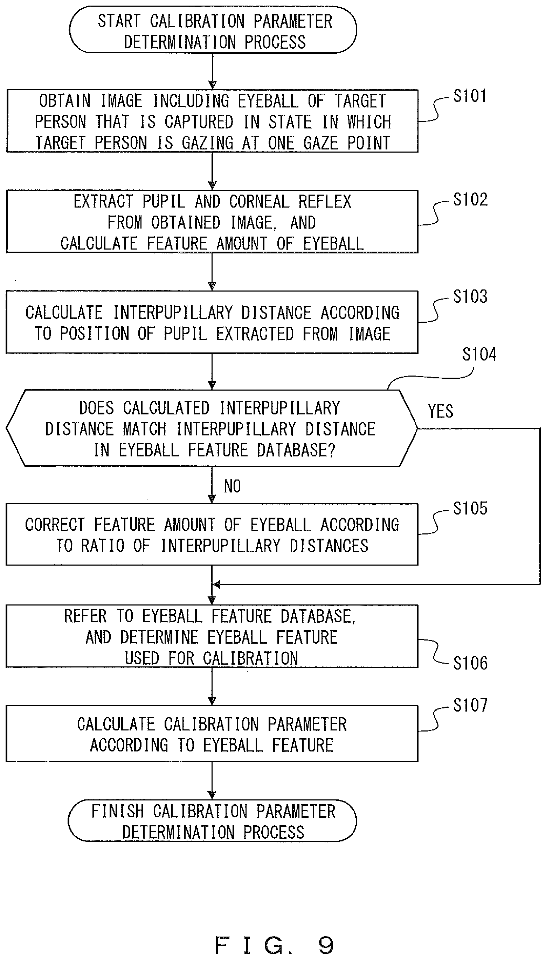

FIG. 9 is a flowchart explaining the content of the calibration parameter determination process according to the first embodiment.

In the calibration parameter determination process (step S1), the line-of-sight detector 1 first obtains an image including the eyeball of the target person that was captured in a state in which the target person was gazing at one gaze point (step S101). The process of step S101 is performed by the image obtaining unit 101 and the gazing instruction unit 102. In step S101, the image obtaining unit 101 first makes the gazing instruction unit 102 perform a process for making the target person gaze at a prescribed gaze point (a first gaze point). The gazing instruction unit 102 outputs, to the output device (the speaker) 4, a sound signal that causes the target person to gaze at any one point (for example, the gaze point PG3) of the gaze points PG1 to PG5 in the eyeball feature database 121, and makes the target person gaze at the gaze point PG3. Meanwhile, the image obtaining unit 101 makes the infrared camera 201 of the line-of-sight sensor 2 capture an image in a state in which the target person is gazing at a prescribed gaze point (the gaze point PG3), and obtains an image captured by the infrared camera 201. Then, the image obtaining unit 101 transmits the obtained image to the feature amount calculator 103.

When the line-of-sight detector 1 obtains an image of the target person, the line-of-sight detector 1 extracts the pupil and a corneal reflex from the obtained image, and calculates a feature amount of the eyeball, by using the feature amount calculator 103 (step S102). The feature amount extraction unit 103 extracts the center of the pupil and the center of the corneal reflex of each of the right eye and the left eye in the image, and calculates a relative position of the center of the pupil viewed from the center of the corneal reflex as a feature amount of the eyeball, similarly to the feature amount extraction unit 703 of the database generator 7. The feature amount calculator 103 extracts a pixel including the center of the pupil and a pixel including the center of the corneal reflex in the image in accordance with a known extraction method in the pupil-corneal reflex method, and calculates a relative position of the center of the pupil viewed from the center of the corneal reflex.

The feature amount calculator 103 transmits the calculated feature amount of the eyeball to the process switching unit 104. The process switching unit 104 switches a transmission destination of the feature amount of the eyeball depending on whether a process performed by the line-of-sight detector 1 is the calibration parameter determination process (step S1) or the line-of-sight detection process (step S2). When the process performed by the line-of-sight detector 1 is the calibration parameter determination process (step S1), the process switching unit 104 transmits the feature amount of the eyeball to the feature amount corrector 105.

Accordingly, when the calibration parameter determination process is performed, the line-of-sight detector 1 calculates an interpupillary distance in the image on the basis of the positions of the pupils extracted from the image, by using the feature amount corrector 105 (step S103). The feature amount corrector 105 calculates an interpupillary distance (a pixel difference) PD on the basis of coordinates representing the position of a pixel including the center of the pupil of the right eye in the obtained image and coordinates representing the position of a pixel including the center of the pupil of the left eye in accordance with Expression (1).

Then, the feature amount corrector 105 determines whether the calculated interpupillary distance PD matches an interpupillary distance in the eyeball feature database 121 (step S104). When the calculated interpupillary distance PD does not match the interpupillary distance in the eyeball feature database 121 (step S104; NO), the feature amount corrector 105 corrects the feature amount of the eyeball according to a ratio of the interpupillary distances (step S105), and transmits the corrected feature amount of the eyeball to the feature determination unit 106. When the calculated interpupillary distance PD matches the interpupillary distance in the eyeball feature database 121 (step S104; YES), the feature amount corrector 105 skips the process of step S105, and transmits the feature amount of the eyeball calculated in step S103 to the feature determination unit 106.

When the processes of step S103 to S105 are finished, the line-of-sight detector 1 refers to the eyeball feature database 121, and determines an eyeball feature used for calibration, by using the feature determination unit 106 (step S106). The feature determination unit 106 refers to a feature amount of the eyeball at the time of gazing at the gaze point specified in step S101 from among feature amounts of the eyeball that are registered in the eyeball feature database 121. The feature determination unit 106 extracts a record including a feature amount of the eyeball that matches the feature amount of the eyeball received from the feature amount corrector 105, and determines a set of feature amounts of the eyeball that is included in the record as an eyeball feature used for calibration. The feature determination unit 106 transmits the determined eyeball feature (a set of feature amounts of the eyeball that is associated with an identifier) to the parameter calculator 107.

When the process of step S106 is finished, the line-of-sight detector 1 calculates a calibration parameter according to the determined eyeball feature by using the parameter calculator 107 (step S107), and determines the calculated calibration parameter as a calibration parameter for the target person. The parameter calculator 107 calculates the calibration parameter according to a known calculation method in the pupil-corneal reflex method.

When the process of step S107 is finished, the parameter calculator 107 transmits the calculated (determined) calibration parameter to the line-of-sight calculator 108, and reports to the process switching unit 104 that a calibration parameter for a current target person has been determined. Consequently, the calibration parameter determination process of step S1 that is performed by the line-of-sight detector 1 is finished.

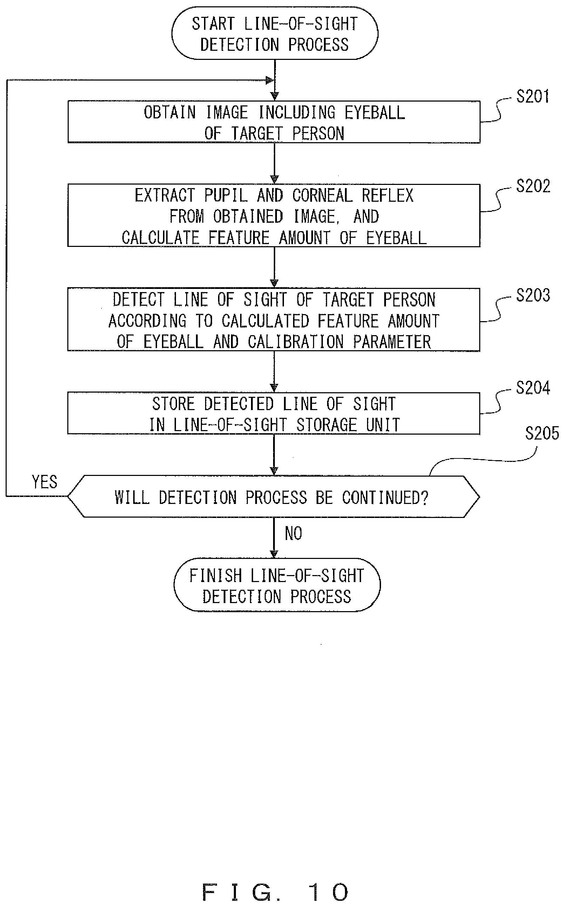

After determining the calibration parameter in the calibration parameter determination process above (steps S101 to S107), the line-of-sight detector 1 performs the processing illustrated in FIG. 10 as the line-of-sight detection process (step S2).

FIG. 10 is a flowchart explaining the content of the line-of-sight detection process.

When the line-of-sight detection process is started, the line-of-sight detector 1 first obtains an image including the eyeball of the target person (step S201). The process of step S201 is performed by the image obtaining unit 101. The image obtaining unit 101 may continue to obtain an image that is periodically captured by the infrared camera 201 after the process of step S101 in FIG. 9 is finished, or may start to obtain an image captured by the infrared camera 201 upon determination of the calibration parameter. The image obtaining unit 101 transmits the obtained image to the feature amount calculator 103.

When the line-of-sight detector 1 obtains the image of the target person, the line-of-sight detector 1 extracts the pupil and a corneal reflex from the obtained image, and calculates a feature amount of the eyeball, by using the feature amount calculator 103 (step S202). In step S202, the feature amount calculator 103 extracts the center of the pupil and the center of the corneal reflex of each of the right eye and the left eye in the image, and calculates a relative position of the center of the pupil viewed from the center of the corneal reflex as a feature amount of the eyeball, similarly to the process of step S102. The feature amount calculator 103 extracts a pixel including the center of the pupil and a pixel including the center of the corneal reflex in the image according to a known extraction method in the pupil-corneal reflex method, and calculates a relative position of the center of the pupil viewed from the center of the corneal reflex.

The feature amount calculator 103 transmits the calculated feature amount of the eyeball to the process switching unit 104. The process switching unit 104 switches a transmission destination of the feature amount of the eyeball depending on whether a process performed by the line-of-sight detector 1 is the calibration parameter determination process (step S1) or the line-of-sight detection process (step S2), as described above. When the line-of-sight detector 1 determines a calibration parameter in the process of step S1, as described above, information reporting that the calibration parameter has been determined is transmitted from a parameter determination unit 107 to the process switching unit 104. Upon receipt of the information from the parameter determination unit 107, the process switching unit 104 determines that the process performed by the line-of-sight detector 1 has been switched to the line-of-sight detection process (step S2). In a case in which the line-of-sight detector 1 is performing the line-of-sight detection process, the process switching unit 104 transmits the feature amount of the eyeball to the line-of-sight calculator 108.

Accordingly, in a case in which the line-of-sight detection process is being performed, the line-of-sight detector 1 calculates a line of sight of the target person on the basis of the calculated feature amount of the eyeball and the calibration parameter by using the line-of-sight calculator 108 (step S203). The line-of-sight calculator 108 calculates the direction, position, and the like of the line of sight of the target person according to a known line-of-sight calculation method in the pupil-corneal reflex method.

When the line-of-sight calculator 108 calculates the line of sight of the target person, the line-of-sight calculator 108 stores the calculated line of sight in the line-of-sight storage unit 122 (step S204).

When the processes of steps S201 to S204 are finished, the line-of-sight detector 1 determines whether the detection process will be continued (step S205). As an example, in a case in which the detection process will not be continued (step S205; NO), such as a case in which the target person operates the input device 3 so as to input information indicating that the detection process will be finished or a case in which the target person moves the infrared camera 201 to the outside of an imaging range of the infrared camera 201, the line-of-sight detector 1 finishes the line-of-sight detection process. In a case in which the detection process will be continued (step S205; YES), the line-of-sight detector 1 repeats the line-of-sight detection process (steps S201 to S204).

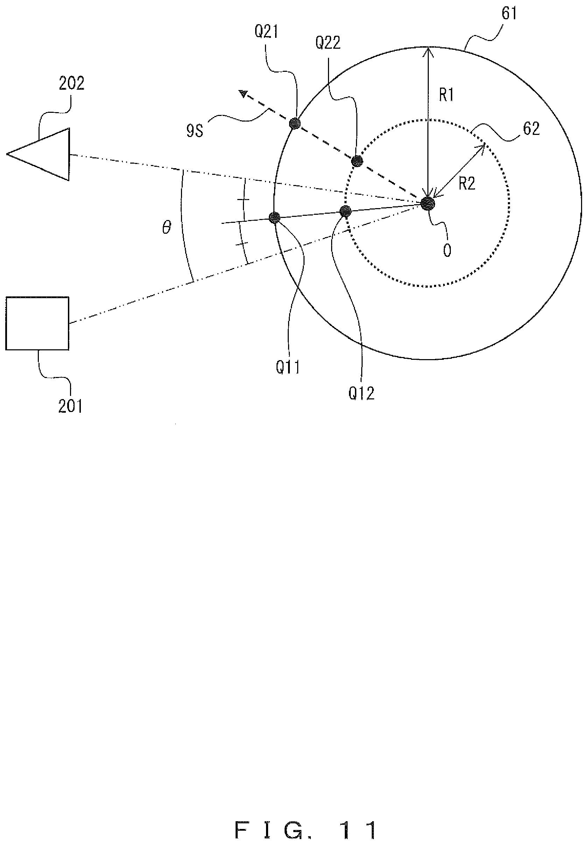

FIG. 11 is a diagram explaining a correlation between the position of the pupil and the position of a corneal reflex, and a corneal radius.

In a case in which a line of sight of the target person is detected according to the pupil-corneal reflex method, as in the line-of-sight detector 1 according to this embodiment, the position of the pupil and the position of a corneal reflex in an image captured by the infrared camera 201, and the corneal radius of the target person are correlated.

As an example, when the centers O of the eyeballs of two eyeballs 61 and 62 having different corneal radiuses overlap each other, as illustrated in FIG. 11, the directions of the centers Q21 and Q22 of the pupils viewed from the centers O of the eyeballs (the directions of the lines of sight 9S) are the same as each other regardless of the corneal radiuses R1 and R2. In addition, the directions of the centers Q11 and Q12 of corneal reflexes viewed from the centers O of the eyeballs are the direction of a line segment that bisects an angle $\theta$ that is formed by a line segment connecting the infrared LED 202 and the centers O of the eyeballs and a line segment connecting the infrared camera 201 and the centers O of the eyeballs, regardless of the corneal radiuses R1 and R2. Accordingly, in a case in which distances from the infrared camera 201 to the centers O of the eyeballs are the same as each other, a distance from the center of a corneal reflex to the center of the pupil for the eyeball 61 having a large corneal radius is longer than a distance for the eyeball 62 having a small corneal radius.

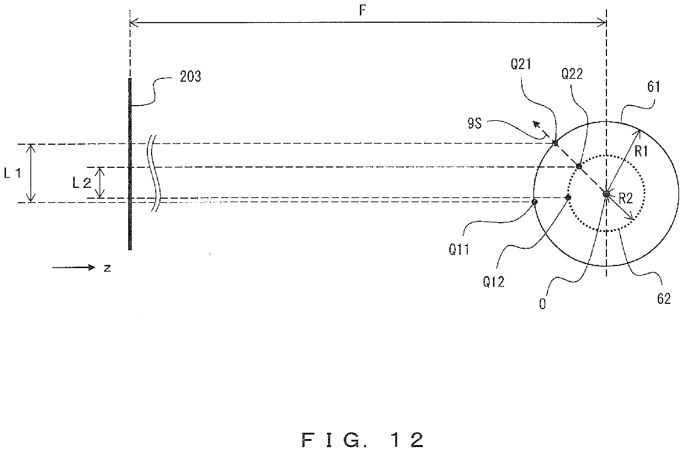

Further, in a real space, a distance from the infrared camera 201 to the centers O of the eyeballs is much longer than the corneal radiuses R1 and R2 of the eyeballs. Therefore, a correlation between a distance from the center of a corneal reflex to the center of the pupil on an imaging plane of the infrared camera 201 and a corneal radius is represented, as illustrated in FIG. 12.

FIG. 12 is a diagram explaining a correlation between the position of the pupil and the position of a corneal reflex on an image, and a corneal radius.

A distance F from an imaging plane 203 of the infrared camera 201 to the centers O of the eyeballs in FIG. 12 is much longer than the corneal radiuses R1 and R2 of the eyeballs 61 and 62. Accordingly, it can be considered that infrared rays that direct from the centers Q11 and Q12 of corneal reflexes to the imaging plane 203 and infrared rays that direct from the centers Q21 and Q22 of the pupils of the eyeballs to the imaging plane 203 are parallel to each other regardless of the corneal radiuses R1 and R2.

Assumed that the directions of the centers Q21 and Q22 of the pupils viewed from the centers O of the eyeballs (the directions of the lines of sight 9S) are the same as each other. In this case, a distance L1 from the center Q11 of the corneal reflex to the center Q21 of the pupil of the eyeball 61 is longer than a distance L2 from the center Q12 of the corneal reflex to the center Q22 of the pupil of the eyeball 62 on the imaging plane 203. As described above, even when a plurality of target persons look in the same direction in the same position viewed from the infrared camera 201, feature amounts of the eyeballs in images have different values from each other according to corneal radiuses (the sizes of the eyeballs) of the respective target persons. Namely, in a case in which the positions in which the infrared camera 201 and the infrared LED 202 are installed are known and a distance from the infrared camera 201 to the eyeball of a target person is a prescribed distance, a position relationship between the center of a corneal reflex and the center of the pupil depends only on a corneal radius.

As an example, in a case in which a distance from the center of a corneal reflex to the center of the pupil on the imaging plane 203 (the image) is L1, it can be considered that a corneal radius of the eyeball of a target person is the same as the corneal radius R1 of the eyeball 61. Accordingly, if a feature amount of the eyeball of a person for which a corneal radius of the eyeball is R1 has been registered in the eyeball feature database 121, a set of feature amounts of the eyeball of the target person can be determined on the basis of a feature amount of the eyeball at the time of gazing at one gaze point and the eyeball feature database 121.

Namely, if a position relationship between the center of a corneal reflex and the center of the pupil at the time when a distance from the infrared camera 201 to the eyeball of a target person is a prescribed distance is known, a corneal radius (the size of the eyeball) of the target person can be estimated according to the position relationship. If the corneal radius of the target person can be estimated, a position relationship between the center of a corneal reflex and the center of the pupil in an image of the eyeball of the target person who is looking in a direction can be estimated from a position relationship in an image of the eyeball of another person. Accordingly, by registering a set of feature amounts of the eyeball at the time when each of a plurality of persons having different sizes of the eyeballs (different corneal radiuses) gazes at a plurality of gaze points in the eyeball feature database 121, a feature amount of the eyeball of a target person can be determined on the basis of one gaze point. Thus, according to this embodiment, a plurality of feature amounts needed to determine a calibration parameter of a line of sight can be determined only by making a target person gaze at one gaze point, and a burden of gazing at a gaze point that is imposed on the target person can be reduced.

Moreover, in the calibration parameter determination process according to this embodiment, a calibration parameter can be calculated on the basis of an image obtained by using a pair of one infrared camera 201 and one infrared LED 202 and the eyeball feature database 121. Thus, according to this embodiment, a plurality of infrared cameras 201 or infrared illumination devices do not need to be installed, and the size of a device in a line-of-sight detection system including the line-of-sight detector 1, the infrared camera 201, and the infrared LED 202 can be suppressed from increasing. Accordingly, a cost in introducing the line-of-sight detection system can be suppressed from increasing. Further, the calibration parameter determination process performed by the line-of-sight detector 1 is a process for extracting, from the eyeball feature database 121, a record (an eyeball feature) including a feature amount that matches a feature amount of the eyeball in an obtained image and determining a calibration parameter on the basis of the extracted eyeball feature. Thus, according to this embodiment, the calibration parameter can be determined without performing complicated processing, and a processing load on the line-of-sight detector 1 can be suppressed from increasing.

A feature amount of the eyeball that is calculated from an image captured by the infrared camera 201 is not limited to a relative position of the center of the pupil viewed from the center of a corneal reflex, and may be a relative position of the center of the corneal reflex viewed from the center of the pupil. Further, a value used to determine whether the feature amount of the eyeball will be corrected is not limited to an interpupillary distance, and may be a distance between corneal reflexes of both eyes.

In addition, prepared information relating to an eyeball feature including a set of feature amounts at the time of gazing at respective gaze points is not limited to the database 121 illustrated in FIG. 2, and the information may be registered (stored) in a storage unit in another mode in which the feature amounts can be discriminated from each other.

Further, the line-of-sight detector 1 according to this embodiment is not limited to a line-of-sight detection system that detects a line of sight of a driver (the target person 9) who drives the vehicle 8 above, and can be applied to various line-of-sight detection systems.

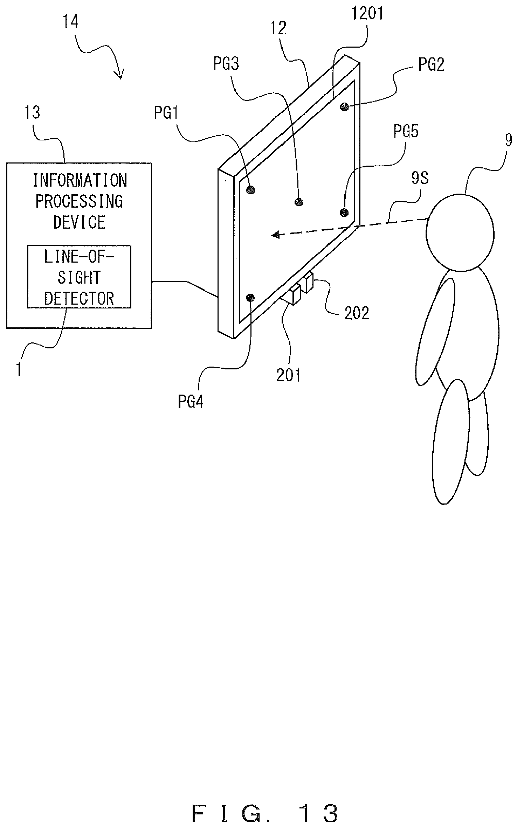

FIG. 13 illustrates another application example of a line-of-sight detector.

The line-of-sight detector 1 according to this embodiment can be applied to a line-of-sight detection system 14 that detects a line of sight 9S of a person 9 who is looking at a display device 12. In this type of line-of-sight detection system 14, the line-of-sight detector 1 can be incorporated into an information processing device 13 that performs various types of processing including, for example, control to display a video, an image, text data, or the like on the display device 12. Further, the database generator 7 can also be incorporated into the information processing device 13, but this is not illustrated in FIG. 13.

A process for generating the eyeball feature database 121 used by the line-of-sight detection system 14 of FIG. 13 may be the same as the processing of FIG. 5, and images of the target person 9 who is gazing at respective gaze points PG1 to PG5 that have been set on a display surface 1201 of the display device 12 are first obtained (steps S11 to S13). At this time, as an example, an image displaying only the gaze point PG1, an image displaying only the gaze point PG2, an image displaying only the gaze point PG3, an image displaying only the gaze point PG4, and an image displaying only the gaze point PG5 are sequentially displayed on the display surface 1201 of the display device 12.

After obtaining a plurality of images, the database generator 7 calculates feature amounts of the eyeballs of the right eye and the left eye, and also calculates an interpupillary distance on the basis of each of the obtained images (steps S14 and S15), and registers them in the eyeball feature database 121 (step S16).

The generated eyeball feature database 121 is stored in the line-of-sight detector 1 (the information processing device 13) via a portable recording medium such as a memory card or an optical disk, or a transmission cable. Also in the line-of-sight detection system 14, the line-of-sight detector 1 that stores the eyeball feature database 121 performs the processing illustrated in FIGS. 8 to 10.

The line-of-sight detection system 14 of FIG. 13 can be used, for example, to input a line of sight. When the target person 9 gazes at a prescribed object displayed on the display surface 1201 of the display device 12, the line-of-sight detector 1 detects a line of sight, and consequently, the information processing device 13 can perform processing associated with the object that the target person 9 is gazing at.

In addition, the line-of-sight detection system 14 of FIG. 13 can be used, for example, to examine which information a target person 9 shows interest in, by using a device that provides various types of information, such as digital signage.

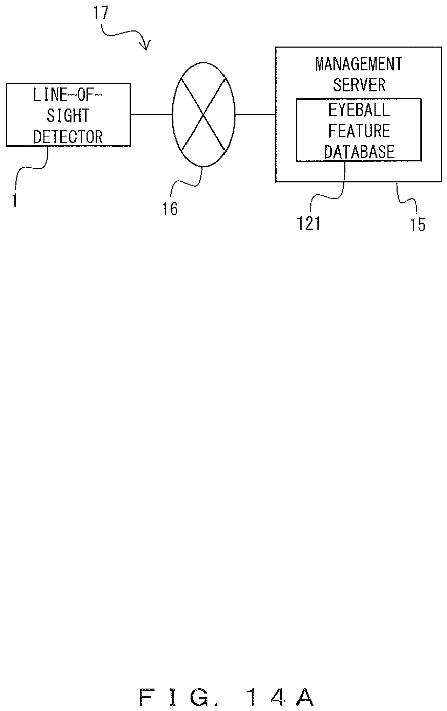

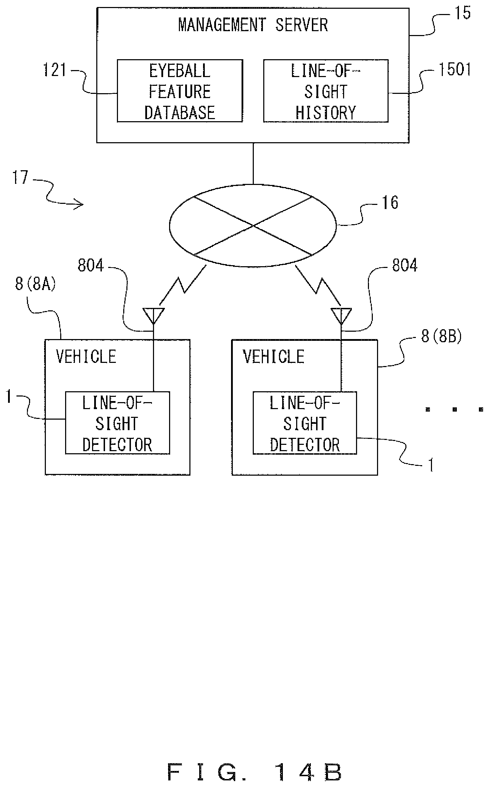

FIGS. 14A and 14B illustrate variations of a line-of-sight detector.

The line-of-sight detector 1 according to this embodiment is not limited to the line-of-sight detection system above, and the line-of-sight detector 1 according to this embodiment can also be applied, for example, to a line-of-sight system 17 in which the eyeball feature database 121 is stored in a management server 15 that is different from the line-of-sight detector 1, as illustrated in FIG. 14A. In this type of line-of-sight system 17, the line-of-sight detector 1 and the management server 15 are communicably connected to each other via a communication network 16 such as the Internet. The line-of-sight sensor 2, the input device 3, and the output device 4 such as a speaker or the display device 12 are connected to the line-of-sight detector 1, but this is not illustrated in FIG. 14A.

Further, the line-of-sight detection system 17 to which the line-of-sight detector 1 is applied may be a system in which line-of-sight detectors 1 installed in respective vehicles 8 (8A and 8B) share the eyeball feature database 121 stored in the management server 15, as illustrated in FIG. 14B. In this type of line-of-sight detection system 17, as an example, a radio communication unit and an antenna 804 are connected to the line-of-sight detector 1 of each of the vehicles 8, and each of the line-of-sight detectors 1 and the eyeball feature database 121 can communicate with each other via the communication network 16. In addition, in the line-of-sight detection system 17 illustrated in FIG. 14B, line-of-sight information of a target person that is detected by each of the line-of-sight detectors 1 can be transferred to a line-of-sight history 1501 of the management server 15. Accordingly, the eyeball feature database 121 can be shared by a plurality of line-of-sight detectors 1, and line-of-sight information of target persons can be managed collectively by the management server 15. Further, in the line-of-sight detection system 17 illustrated in FIG. 14B, as an example, the calibration parameter generation process above may be performed on the management server 15, but this is not illustrated. In this case, the line-of-sight detector 1 transmits, for example, an image captured by the infrared camera 201 and vehicle type information of the vehicle 8 installed with the line-of-sight detector 1 to the management server 15, and makes the management server 15 calculate a calibration parameter. Then, the line-of-sight detector 1 obtains the calibration parameter from the management server 15, and calculates a line of sight of a driver (a target person 9) according to the calibration parameter.

In addition, the line-of-sight detection systems illustrated in FIGS. 6A, 13, 14A, and 14B are application examples of the line-of-sight detector 1 according to this embodiment. Namely, the line-of-sight detector 1 is not limited to the line-of-sight detection system above, and can be applied to various line-of-sight detection systems using the pupil-corneal reflex method. As an example, the line-of-sight detector 1 can be applied to a line-of-sight detection system in which a shop or the like collects information indicating, for example, which commodity of the commodities displayed in the shop a target person (a customer) is interested in.

Further, the functional configuration of the line-of-sight detector 1 illustrated in FIG. 1 and processing performed by the line-of-sight detector 1 are examples. The line-of-sight detector 1 according to this embodiment may start the calibration parameter determination process, for example, when a human detection sensor or the like detects that a target person 9 stops in a prescribed position. In a case in which the line-of-sight detector 1 according to this embodiment is installed in the vehicle 8, as an example, the calibration parameter determination process may be started when a target person 9 (a driver) starts the engine, and the line-of-sight detection process may be finished when the target person 9 stops the engine.

<Second Embodiment>

In this embodiment, a processing method of a line-of-sight detector 1 for correcting a feature mount of the eyeball in consideration of the orientation of a face of a target person and determining a calibration parameter is described.

A functional configuration of the line-of-sight detector 1 according to this embodiment may be the configuration illustrated in FIG. 1. In addition, an eyeball feature database 121 stored in the line-of-sight detector 1 according to this embodiment may have, for example, the configuration illustrated in FIG. 2. However, in generating the eyeball feature database 121 according to this embodiment, an image in which a person is gazing at a gaze point in a state in which a face is facing an infrared camera 201 is obtained, and a feature amount of the eyeball and an interpupillary distance that are calculated from the image are registered in the eyeball feature database 121.

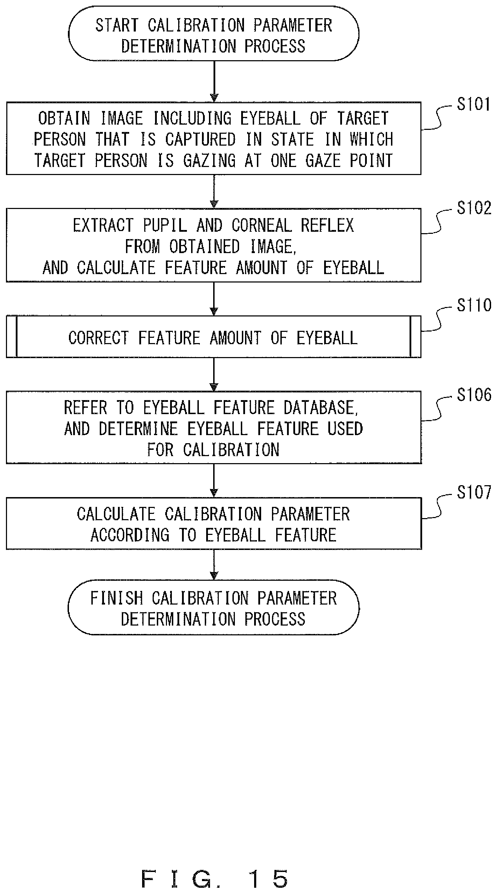

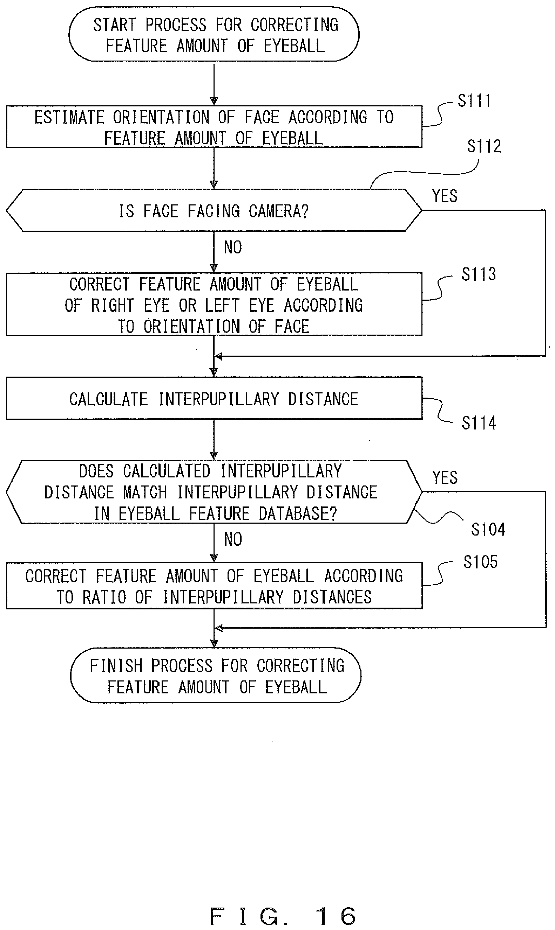

The line-of-sight detector 1 according to this embodiment performs the calibration parameter determination process (step S1) and the line-of-sight detection process (step S2) illustrated in FIG. 8. The line-of-sight detector 1 according to this embodiment performs the processing illustrated in FIGS. 15 and 16 as the calibration parameter determination process.

FIG. 15 is a flowchart explaining the content of a calibration parameter determination process according to the second embodiment. FIG. 16 is a flowchart explaining the content of a process for correcting a feature amount of the eyeball. The same step number (for example, S101, S102, or the like) as the number in FIG. 9 is assigned to each of the blocks in which the same process as the process in the flowchart of FIG. 9 is performed from among processing blocks in the flowcharts of FIGS. 15 and 16.

In the calibration parameter determination process according to this embodiment, the line-of-sight detector 1 first obtains an image including the eyeball of a target person that was captured in a state in which the target person was gazing at one gaze point (step S101). The process of step S101 is performed by the image obtaining unit 101 and the gazing instruction unit 102. In step S101, the image obtaining unit 101 and the gazing instruction unit 102 perform the respective processes described in the first embodiment. The gazing instruction unit 102 makes the target person gaze at one gaze point by using the output device 4. Meanwhile, the image obtaining unit 101 makes the infrared camera 201 capture an image including the eyeball of the target person who is gazing at the gaze point, and obtains the captured image. Further, the image obtaining unit 101 transmits the obtained image to the feature amount calculator 103.

When the line-of-sight detector 1 obtains the image of the target person, the line-of-sight detector 1 extracts the pupil and a corneal reflex from the obtained image, and calculates a feature amount of the eyeball, by using the feature amount calculator 103 (step S102). The feature amount calculator 103 extracts a pixel including the center of the pupil and a pixel including the center of the corneal reflex in each of the right eye and the left eye in the image, and calculates a relative position of the center of the pupil viewed from the center of the corneal reflex as a feature amount of the eyeball, as described in the first embodiment.

The feature amount calculator 103 transmits the calculated feature amount of the eyeball to the process switching unit 104. In a case in which a process performed by the line-of-sight detector 1 is the calibration parameter determination process, the process switching unit 104 transmits the feature amount of the eyeball to the feature amount corrector 105, as described in the first embodiment.

Then, the line-of-sight detector 1 performs a process for correcting the feature amount of the eyeball (step S110) by using the feature amount corrector 105. In step S110, the feature amount corrector 105 first estimates the orientation of a face of the target person in the image on the basis of the feature amount of the right eye and the feature amount of the left eye. In a case in which the estimated orientation of the face does not face the infrared camera 201, the feature amount corrector 105 corrects the feature amounts of the right eye and the left eye so as to be feature amounts at the time when the orientation of the face is facing the infrared camera 201. Then, the feature amount corrector 105 performs the processes of steps S103 to S105 described in the first embodiment, namely, a process for correcting a feature amount of the eyeball in a case in which an interpupillary distance on the image is different from an interpupillary distance in the eyeball feature database 121. The feature amount corrector 105 transmits the corrected feature amount of the eyeball to the feature determination unit 106. When the feature amount of the eyeball is not corrected in step S110, the feature amount corrector 105 transmits the feature amount of the eyeball calculated by the feature amount calculator 103 to the feature determination unit 106.

When the process of step S110 is finished, the line-of-sight detector 1 refers to the eyeball feature database 121, and determines an eyeball feature used for calibration, by using the feature determination unit 106 (step S106). The feature determination unit 106 refers to a feature amount of the eyeball at the time of gazing at the gaze point specified in step S101 from among the feature amounts of the eyeballs registered in the eyeball feature database 121.

Then, the feature determination unit 106 determines a set of feature amounts including the same feature amount as the feature amount of the eyeball received from the feature amount corrector 105 to be an eyeball feature used for calibration. The feature determination unit 106 transmits the determined eyeball feature (a set of feature amounts of the eyeballs that is associated with an identifier) to the parameter calculator 107.

When the process of step S106 is finished, the line-of-sight detector 1 calculates a calibration parameter according to the determined eyeball feature by using the parameter calculator 107 (step S107). The parameter calculator 107 calculates a calibration parameter according to a known calculation method in the pupil-corneal reflex method, and determines the calculated calibration parameter to be a calibration parameter for a current target person. When the process of step S107 is finished, the parameter calculator 107 transmits the determined calibration parameter to the line-of-sight calculator 108, and reports to the process switching unit 104 that the calibration parameter for the current target person has been determined. Consequently, the calibration parameter determination process performed by the line-of-sight detector 1 is finished.

The process of step S110 for correcting a feature amount of the eyeball that is performed by the feature amount corrector 105 is described next with reference to FIG. 16.

In the process of step S110, the feature amount corrector 105 first estimates the orientation of a face of a target person in accordance with a feature amount of the eyeball (step S111), and determines whether the face of the target person is facing the infrared camera 201 (step S112). In step S111, the feature amount corrector 105 estimates the orientation of the face in accordance with, for example, a magnitude relationship between a feature amount of the right eye and a feature amount of the left eye. When the feature amount of the right eye is almost the same as the feature amount of the left eye, the feature amount corrector 105 estimates that the face of the target person is facing the infrared camera 201. When an absolute value of a difference between the feature amount of the right eye and the feature amount of the left eye is greater than or equal to a threshold, the feature amount corrector 105 estimates that the orientation of the face is inclined in a rightward or leftward direction.

As a result of estimating the orientation of the face in step S111, when the face is not facing the infrared camera 201 (step S112, NO), the feature amount corrector 105 corrects the feature amount of the eyeball of the right eye or the left eye in accordance with the orientation of the face (step S113). In S113, the feature amount corrector 105 corrects a center position of a corneal reflex and a center position of the pupil of the right eye or the left eye in accordance with the orientation of the face estimated in step S111 in such a way that the feature amount of the right eye becomes almost the same as the feature amount of the left eye. After step S113, the feature amount corrector 105 calculates an interpupillary distance in accordance with the center position of the pupil of the right eye and the center position of the pupil of the left eye after correction (step S114).

When the face is facing the infrared camera 201 (step S112; YES), the feature amount corrector 105 skips the process of step S113, and performs the process of step S114. When the process of step S113 is skipped, the feature amount corrector 105 calculates an interpupillary distance in accordance with the center position of the pupil of the right eye and the center position of the pupil of the left eye that were used to estimate the orientation of the face.

When the interpupillary distance is calculated, the feature amount corrector 105 determines whether the calculated interpupillary distance matches an interpupillary distance in the eyeball feature database 121 (step S104). When the interpupillary distances do not match each other (step S104; NO), the feature amount corrector 105 corrects the feature amount of the eyeball accordance with a ratio of the interpupillary distances (step S105). Processing performed by the feature amount corrector 105 in step S105 is as described in the first embodiment. After step S105, the feature amount corrector 105 transmits the corrected feature amount of the eyeball to the feature determination unit 106, and finishes the process for correcting the feature amount of the eyeball.

When the interpupillary distances match each other (step S104; YES), the feature amount corrector 105 skips the process of step S105, transmits the feature amount of the eyeball to the feature determination unit 106, and finishes the process for correcting the feature amount of the eyeball. When the interpupillary distances match each other, the feature amount corrector 105 outputs, to the feature determination unit 106, the feature amount of the eyeball extracted by the feature amount calculator 103 or the feature amount of the eyeball corrected in step S113.

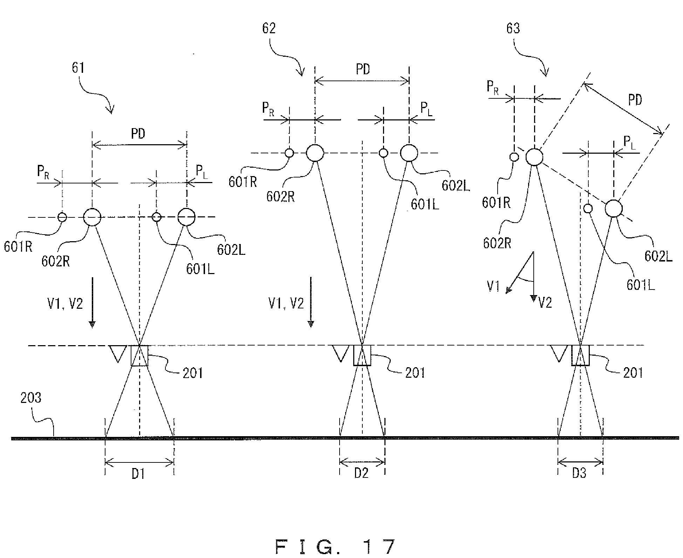

FIG. 17 is a diagram explaining a relationship between the orientation of a face and a feature amount of the eyeball.

A model 61 on a left-hand side of FIG. 17 illustrates a feature amount of the eyeball and an interpupillary distance on the imaging plane 203 in a case in which the orientation V1 of a face is a direction of the infrared camera 201 (namely, the face is facing the infrared camera 201), and the direction V2 of a line of sight is the direction of the infrared camera 201. In this case, a distance $P_L$ from the center of a corneal reflex 601R to the center of the pupil 602R of the right eye viewed from the infrared camera 201 (the imaging plane 203) and a distance $P_R$ from the center of a corneal reflex 601L to the pupil 602L of the left eye have almost the same value ($P_R \approx P_L$).

A model 62 in the middle of FIG. 17 illustrates a feature amount of the eyeball and an interpupillary distance on an imaging plane in a case in which the orientation V1 of the face is a direction of the infrared camera 201, and the direction V2 of the line of sight is the direction of the infrared camera 201. Accordingly, also in the case of the model 62 of FIG. 17, the distance $P_L$ from the corneal reflex to the pupil of the right eye and the distance $P_R$ from the corneal reflex to the pupil of the left eye have almost the same value ($P_R \approx P_L$).

As described above, when the face is facing the infrared camera 201, the distance $P_R$ from the corneal reflex 601R to the pupil 602R of the right eye and the distance $P_L$ from the corneal reflex 601L to the pupil 602L of the left eye have almost the same value ($P_R \approx P_L$).

However, in the model 62 in the middle of FIG. 17, a distance from the infrared camera 201 (the imaging plane 203) to the eyeball (the face) is longer than a distance from the infrared camera 201 to the eyeball in the model 61 of FIG. 17. Accordingly, even when actual interpupillary distances PD in the model 61 and the model 62 of FIG. 17 are the same as each other, an interpupillary distance D2 on the imaging plane 203 in the model 62 is shorter than an interpupillary distance D1 on the imaging plane 203 in the model 61 (D1 > D2).

On the other hand, a model 63 on a right-hand side of FIG. 17 illustrates a feature amount of the eyeball and an interpupillary distance on the imaging plane 203 in a case in which the orientation V1 of the face is different from the direction of the infrared camera 201, and the direction V2 of the line of sight is the direction of the infrared camera 201. In the model 63 of FIG. 17, a distance from the infrared camera 201 to the right eye is longer than a distance from the infrared camera 201 to the left eye. Accordingly, a distance $P_R$ from the center of the corneal reflex 601R to the center of the pupil 602R of the right eye is shorter than a distance $P_L$ from the center of the corneal reflex 601L to the center of the pupil 602L of the left eye ($P_R < P_L$). Further, in the model 63 of FIG. 17, the orientation V1 of the face of the target person is different from the direction of the infrared camera 201. Therefore, an interpupillary distance D3 viewed from the infrared camera 201 (the imaging plane 203) is shorter than an interpupillary distance calculated according to an actual interpupillary distance PD and distances from the infrared camera 201 (the imaging plane 203) to the eyeballs. On the other hand, a feature amount of the eyeball and an interpupillary distance in the eyeball feature database 121 used according to this embodiment are values that are calculated according to an image captured in a state in which the face is facing the infrared camera 201. Accordingly, in a case in which the face is not facing the infrared camera 201, the feature amount of the eyeball calculated from the image needs to be corrected to a feature amount in a state in which the face is facing the infrared camera 201 in order to correctly compare the calculated feature amount with the feature amount of the eyeball in the eyeball feature database 121.

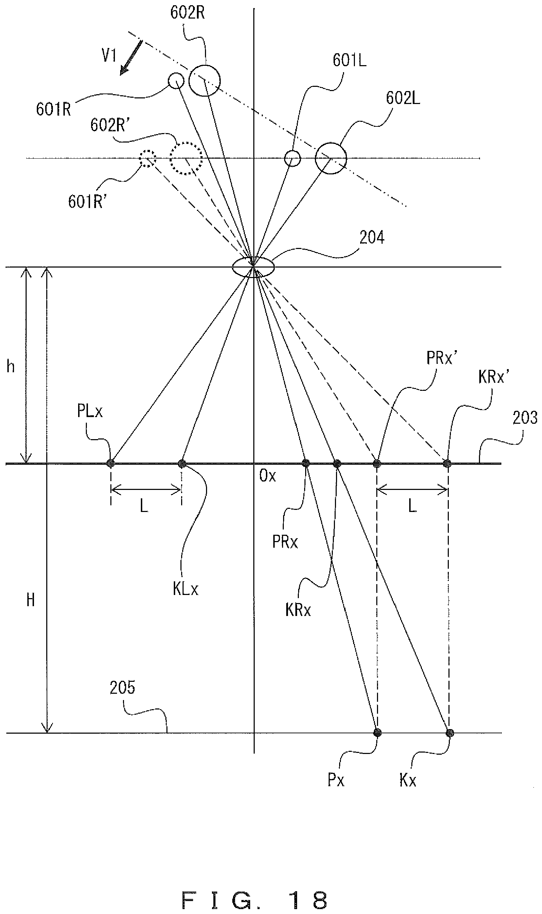

FIG. 18 is a diagram explaining a method for correcting a feature amount of the eyeball in a case in which a face is not facing a camera.

FIG. 18 illustrates a feature amount of the eyeball in a case in which a distance from the imaging plane 203 to a right eye is longer than a distance from the imaging plane 203 to a left eye, as an example of a case in which a face is not facing the infrared camera 201. In this case, a distance from the center KRx of the corneal reflex 601R of the right eye to the center PRx of the pupil 602R on the imaging plane 203 is shorter than a distance L from the center KLx of the corneal reflex 601L to the center PLx of the pupil 602L on the imaging plane 203. Therefore, the feature amount corrector 105 corrects the position of the corneal reflex 601R and the position of the pupil 602R in such a way that the distance from the center of the corneal reflex 601R to the center of the pupil 602R of the right eye on the imaging plane 203 matches the distance L of the left eye (step S113 of FIG. 16). Namely, in step S113, the feature amount corrector 105 calculates points KRx' and PRx' on the imaging plane 203 that satisfy Expression (2) below.

$$\text{KRx}' - \text{PRx}' = \text{KLx} - \text{PLx} \qquad (2)$$

In Expression (2), the point KRx' and the point PRx' are respectively the center of a corneal reflex 601R' and the center of the pupil 602R' of a virtual right eye on the imaging plane 203 in a case in which a target person makes a face be facing the infrared camera 201 by using the position of the left eye as a reference.

In a case in which the position of the corneal reflex 601R and the position of the pupil 602R of the right eye are corrected, a virtual plane 205 on which a distance from the center Px of the corneal reflex 601R to the center Kx of the pupil 602R of the right eye matches the distance L of the left eye is set, as illustrated in FIG. 18. Assume that a distance from a lens 204 of the infrared camera 201 to the imaging plane 203 is a distance h, and that a distance from the lens 204 to the virtual plane 205 is a distance H. A ratio h:H of the two distances h and H is expressed according to Expression (3-1) below.

$$h:H = (\text{KRx} - \text{PRx}):(\text{KLx} - \text{PLx}) \qquad (3-1)$$

Accordingly, the distance H from the lens 204 to the virtual plane 205 is calculated according to Expression (3-2) below.

$$H = h \times \{(KLx - PLx)/(KRx - PRx)\} \qquad (3-2)$$

The ratio h:H of the two distances h and H is expressed according to Expression (4-1) below by using the center Ox of the imaging plane 203, a position KRx before correction of the corneal reflex 601R of the right eye on the imaging plane 203, and the position KRx' of the corneal reflex 601R' of the virtual right eye on the imaging plane 203.

$$h:H = (KRx - Ox):(KRx' - Ox) \qquad (4-1)$$

Accordingly, if the distance H from the lens 204 to the virtual plane 205 is known, the position KRx' of the corneal reflex 601R' of the virtual right eye on the imaging plane 203 can be calculated according to Expression (4-2) below.

$$(KRx' - Ox) = (KRx - Ox) \times (H/h) \qquad (4-2)$$

In addition, the ratio h:H of the two distances h and H can be expressed according to Expression (5-1) below by using the center Ox of the imaging plane 203, a position PRx before correction of the pupil 602R of the right eye on the imaging plane 203, and the position PRx' of the pupil 602R' of the virtual right eye on the imaging plane 203.

$$h:H = (PRx - Ox):(PRx' - Ox) \qquad (5-1)$$

Accordingly, if the distance H from the lens 204 to the virtual plane 205 is known, the position PRx' of the pupil 602R' of the virtual right eye on the imaging plane 203 can be calculated according to Expression (5-2) below.

$$(PRx' - Ox) = (PRx - Ox) \times (H/h) \qquad (5-2)$$

In a case in which the feature amount of the right eye is corrected according to Expressions (3-2), (4-2), and (5-2), the feature amount corrector 105 calculates an interpupillary distance on the basis of a corrected feature amount of the eyeball, namely, a position relationship between the point PLx and the point PRx' on the imaging plane 203 (step S114). When the calculated interpupillary distance does not match an interpupillary distance in the eyeball feature table (step S114; NO), the feature amount corrector 105 corrects the feature amount of the eyeball again according to a ratio of the interpupillary distances (step S105).

By performing the process above for correcting the feature amount of the eyeball, a feature amount of the eyeball calculated from an image becomes a feature amount in which the orientation of a face and an interpupillary distance match the orientation of a face and an interpupillary distance of a person at the time of generating the eyeball feature database 121. Thus, according to this embodiment, a feature amount of the eyeball (a distance between a corneal reflex and the pupil) according to the orientation of a face and an incorrect eyeball feature due to a change in an interpupillary distance can be suppressed from being extracted, and the accuracy of detection of a line of sight can be enhanced.

FIGS. 15 and 16 are an example of the calibration parameter determination process performed by the line-of-sight detector 1 according to this embodiment. The calibration parameter determination process performed by the line-of-sight detector 1 according to this embodiment can be changed appropriately. The procedure of the calibration parameter determination process according to this embodiment is not limited to the procedure illustrated in FIGS. 15 and 16, and the content of some processes may be changed.

In addition, prepared information relating to an eyeball feature including a set of feature amounts of the eyeballs at the time of gazing at respective gaze points is not limited to an eyeball feature database, as described above, and the information may be registered (stored) in a storage unit in another mode in which the feature amounts can be discriminated from each other.

Further, the line-of-sight detector 1 according to this embodiment can be applied to various line-of-sight detection systems that detect a line of sight of a target person from an image, similarly to the line-of-sight detector 1 according to the first embodiment.

<Third Embodiment>

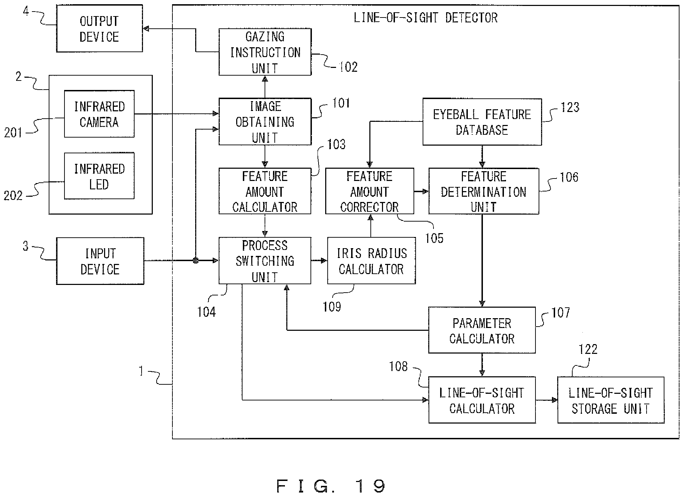

FIG. 19 illustrates a functional configuration of a line-of-sight detector according to a third embodiment.

As illustrated in FIG. 19, a line-of-sight detector 1 according to this embodiment includes an image obtaining unit 101, a gazing instruction unit 102, a feature amount calculator 103, a process switching unit 104, a feature amount corrector 105, a feature determination unit 106, a parameter calculator 107, and a line-of-sight calculator 108. The line-of-sight detector 1 also includes an eyeball feature database 123 and a line-of-sight storage unit 122. The line-of-sight detector 1 further includes an iris radius calculator 109.

Respective functions of the image obtaining unit 101, the gazing instruction unit 102, the feature amount calculator 103, the process switching unit 104, the parameter calculator 107, and the line-of-sight calculator 108 in the line-of-sight detector 1 according to this embodiment are as described in the first embodiment.

The iris radius calculator 109 extracts the iris of the eyeball in an image that the image obtaining unit 101 obtains from the infrared camera 201, and extracts an iris radius.

The feature amount corrector 105 corrects the feature amount of the eyeball and the iris radius on the basis of an interpupillary distance in the image obtained by the image obtaining unit 101 and an interpupillary distance registered in the eyeball feature database 123.

The feature determination unit 106 determines an eyeball feature of a target person (a set of feature amounts of the eyeball) on the basis of the feature amount of the eyeball and the iris radius in the obtained image (or the feature amount of the eyeball and the iris radius after correction) and the eyeball feature database 123.

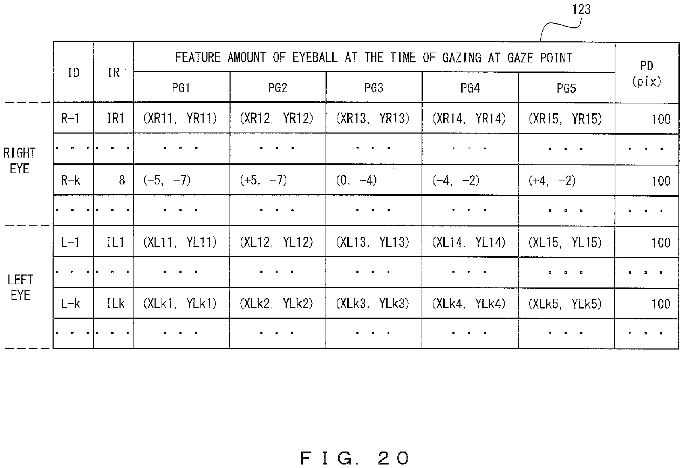

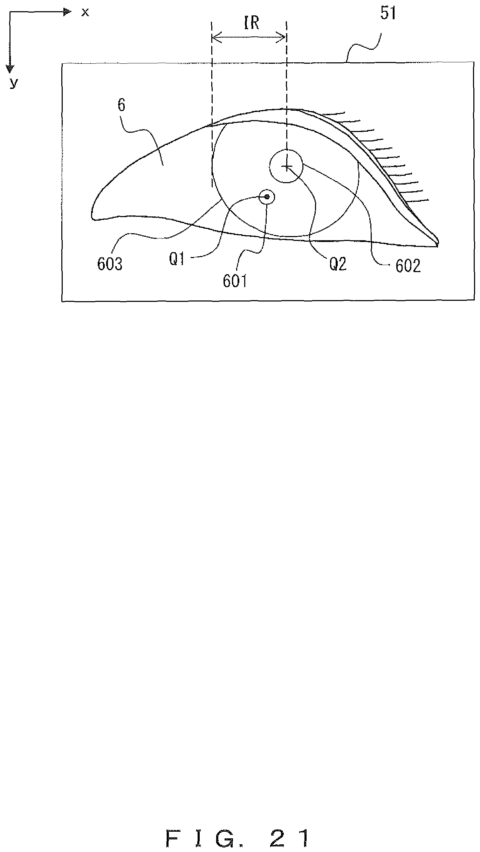

FIG. 20 illustrates an example of an eyeball feature database according to the third embodiment. FIG. 21 is a diagram explaining a feature amount of the eyeball and an iris radius.

As illustrated in FIG. 20, the eyeball feature database 123 according to this embodiment includes an identifier (ID), an iris radius (IR), a feature amount of the eyeball at the time of gazing at a gaze point (PG1 to PG5), and an interpupillary distance (PD). From among them, the identifier, the feature amount of the eyeball, and the interpupillary distance respectively represent the same values in the eyeball feature database 121 described in the first embodiment.

The iris radius (IR) is a radius in an image of the iris of each target person that is extracted from the image when the eyeball feature database 123 is generated.

The eyeball feature database 123 of FIG. 20 can be generated, for example, by the database generator 7 of FIG. 4. In a case in which the eyeball feature database 123 is generated by the database generator 7, as an example, the feature amount calculator 703 calculates a feature amount of the eyeball (a relative position of the center of the pupil viewed from the center of a corneal reflex), and also calculates an iris radius. The registration unit 705 assigns one identifier to a set of the feature amount of the eyeball, the iris radius, and an interpupillary distance calculated by the distance calculator 704, and registers them as one record in the eyeball feature database 123.

The feature amount calculator 703 extracts an annular region 603 surrounding the pupil 602 as the iris from a partial region 51 of the image, and specifies an average distance (the number of pixels) from the center Q2 of the pupil 602 to an outer periphery of the annular region 603 to be the iris radius IR, as illustrated in FIG. 21, for example. In addition, the feature amount of the eyeball extracted by the feature amount calculator 703 is as described in the first embodiment, and the feature amount of the eyeball is assumed to be a value representing a relative position of a pixel including the center Q2 of the pupil 602 viewed from a pixel including the center Q1 of the corneal reflex 601 in the image.

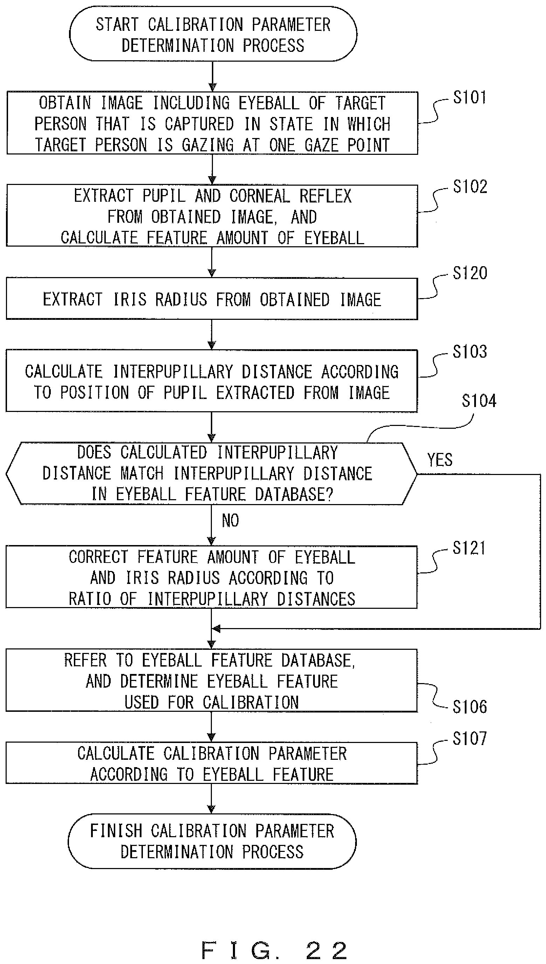

When the line-of-sight detector 1 according to this embodiment starts an operation, the line-of-sight detector 1 performs the calibration parameter determination process (step S1) and the line-of-sight detection process (step S2) illustrated in FIG. 8. The line-of-sight detector 1 according to this embodiment performs the processing illustrated in FIG. 22 as the calibration parameter determination process.

FIG. 22 is a flowchart explaining the content of the calibration parameter determination process according to the third embodiment. From among processing blocks in the flowchart of FIG. 22, each of the blocks in which the same processes as those in the flowchart of FIG. 9 are performed is assigned the same step number (for example, S101, S102, or the like) of FIG. 9.

In the calibration parameter determination process according to this embodiment, the line-of-sight detector 1 first obtains an image including the eyeball of a target person that is captured in a state in which the target person is gazing at one gaze point (step S101). The process of step S101 is performed by the image obtaining unit 101 and the gazing instruction unit 102. In step S101, the image obtaining unit 101 and the gazing instruction unit 102 perform the respective processes described in the first embodiment. The gazing instruction unit 102 makes the target person gaze at one gaze point by using the output device 4. Meanwhile, the image obtaining unit 101 makes the infrared camera 201 capture an image including the eyeball of the target person who is gazing at the gaze point, and obtains the captured image. Further, the image obtaining unit 101 transmits the obtained image to the feature amount calculator 103.

When the line-of-sight detector 1 obtains the image of the target person, the line-of-sight detector 1 extracts the pupil and a corneal reflex from the obtained image, and calculates a feature amount of the eyeball, by using the feature amount calculator 103 (step S102). The feature amount calculator 103 extracts a pixel including the center of the pupil and a pixel including the center of the corneal reflex in each of the right eye and the left eye in the image, and calculates a relative position of the center of the pupil viewed from the center of the corneal reflex as a feature amount of the eyeball, as described in the first embodiment.

The feature amount calculator 103 transmits the calculated feature amount of the eyeball and the obtained image to the process switching unit 104. In a case in which a process performed by the line-of-sight detector 1 is the calibration parameter determination process (step S1), a process selecting unit 104 transmits the feature amount of the eyeball and the image to the iris radius calculator 109.

Then, the line-of-sight detector 1 extracts the iris from the image, and calculates an iris radius by using the iris radius calculator 109 (step S120). The iris radius calculator 109 extracts, for example, an annular region surrounding the pupil as the iris from each of the regions in which the right eye and the left are photographed in the image. The iris radius calculator 109 calculates an average value of a distance between the center of the pupil and an outer periphery of the extracted annular region, and specifies the average value of the distance to be an iris radius. An iris radius calculator 110 transmits the calculated iris radius and the feature amount of the eyeball calculated by the feature amount calculator 103 to the feature amount corrector 105.

After step S120, the line-of-sight detector 1 calculates an interpupillary distance on the basis of the positions of the pupils extracted from the image by using the feature amount corrector 105 (step S103). The feature amount corrector 105 calculates an interpupillary distance (a pixel difference) PD on the basis of the position of the pixel including the center of the pupil of the right eye and the position of the pixel including the center of the pupil of the left eye in the image, the pixels being extracted by the feature amount calculator 103.

Then, the feature amount corrector 105 determines whether the calculated interpupillary distance PD matches an interpupillary distance in the eyeball feature database 123 (step S104). When the calculated interpupillary distance PD does not match the interpupillary distance in the eyeball feature database 123 (step S104; NO), the feature amount corrector 105 corrects the feature amount of the eyeball and the iris radius according to a ratio of the interpupillary distances (step S121). When the feature amount of the eyeball and the iris radius are corrected, the feature amount corrector 105 transmits the feature amount of the eyeball and the iris radius after correction to the feature determination unit 106. When the calculated interpupillary distance PD matches the interpupillary distance in the eyeball feature database 123 (step S104; YES), the feature amount corrector 105 transmits the feature amount of the eyeball calculated in step S103 and the iris radius calculated in step S120 to the feature determination unit 106.

when the feature amount of the eyeball and the iris radius are transmitted to the feature determination unit 106, the line-of-sight detector 1 refers to the eyeball feature database 123, and determines an eyeball feature used for calibration by using the feature determination unit 106 (step S106). The feature determination unit 106 refers to a feature amount of the eyeball at the time of gazing at the gaze point specified in step S101 from among feature amounts of the eyeball, and the iris radius (IR) that have been registered in the eyeball feature database 123. The feature determination unit 106 determines, as an eyeball feature used for calibration, a set of feature amounts included in a record in which a combination of the feature amount of the eyeball and the iris radius in the eyeball feature database 123 is the same as a combination of the feature amount of the eyeball and the iris radius that are received from the feature amount corrector 105, or a record in which the combination of the feature amount of the eyeball and the iris radius in the eyeball feature database 123 is the closest to the combination of the feature amount of the eyeball and the iris radius that are received from the feature amount corrector 105. The feature determination unit 106 transmits the determined eyeball feature (a set of feature amounts of the eyeball that is associated with an identifier) to the parameter calculator 107.

When the process of step S106 is finished, the line-of-sight detector 1 calculates a calibration parameter on the basis of the determined eyeball feature by using the parameter calculator 107 (step S107). The parameter calculator 107 calculates a calibration parameter according to a known calculation method in the pupil-corneal reflex method, and determines the calculated calibration parameter to be a calibration parameter for a current target person. When the process of step S107 is finished, the parameter calculator 107 transmits the determined calibration parameter to the line-of-sight calculator 108, and reports to the process switching unit 104 that the calibration parameter for the current target person has been determined. Consequently, the calibration parameter determination process performed by the line-of-sight detector 1 is finished.

After determining a calibration parameter by performing the calibration parameter determination process illustrated in FIG. 22, the line-of-sight detector 1 according to this embodiment performs, for example, the processing illustrated in FIG. 10 as the line-of-sight detection process.

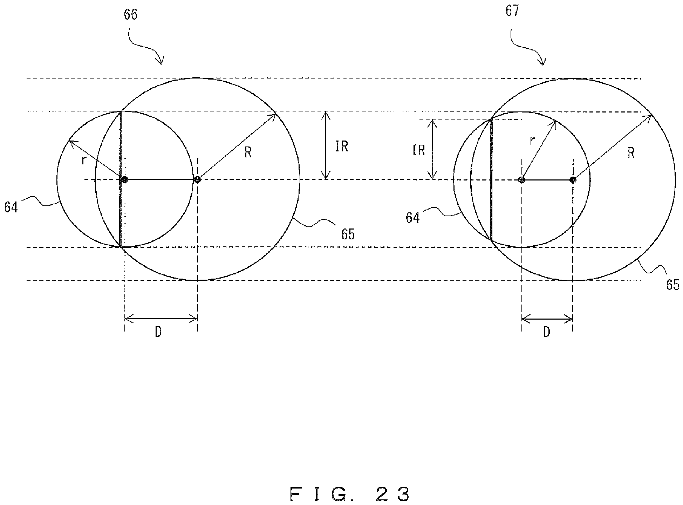

FIG. 23 is a diagram explaining a correlation between an iris radius and the shape of the eyeball.

As a main parameter in an eyeball model, the radius R of the eyeball 65 that controls the rotation of an eye itself, and a distance D between the center of a ball 64 that forms the cornea and the center of the eyeball 65, in addition to the radius (a corneal radius r) of the ball 64 that forms the cornea, are considered, for example, as illustrated in FIG. 23.

In addition, in a case in which the corneal radius r is known in an eyeball model, the distance D between the center of the ball 64 that forms the cornea and the center of the eyeball 65 is correlated with an iris diameter I (the iris radius IR).

As an example, a distance D in an eyeball model 66 on a left-hand side of FIG. 23 is longer than a distance D in an eyeball model 67 on a right-hand side of FIG. 23. At this time, an iris diameter I in the eyeball model 66 is greater than an iris diameter I in the eyeball model 67. As described above, in a case in which the corneal radius r is the same but the iris diameter I (the iris radius IR) is different, a distance between the cornea and the center of the eyeball 65 is different, therefore a difference is generated in a distance between the center of a corneal reflex and the center of the pupil (see, for example, FIGS. 11 and 12). Accordingly, by using the iris radius in addition to a position relationship between the center of the corneal reflex and the center of the pupil, as in this embodiment, eyeball features can be finely classified, and an optimum eyeball feature for a target person can be selected.

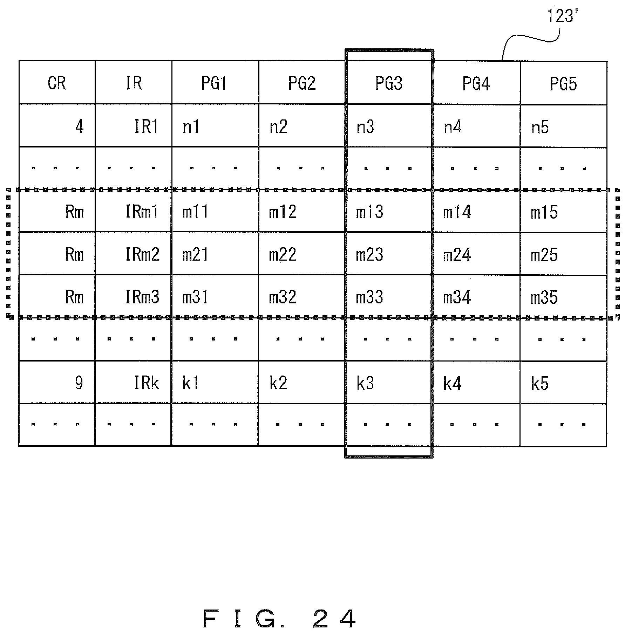

FIG. 24 is a diagram explaining an example of a method for determining an eyeball feature according to the third embodiment.

FIG. 24 illustrates a simplified eyeball feature database 123' according to the third embodiment. A corneal radius (CR), in addition to a feature amount of the eyeball (PG1 to PG5) and an iris radius (IR), is illustrated in the eyeball feature database 123' of FIG. 24.

In an eyeball model, even when the corneal radius is constant, an iris diameter I (an iris radius) changes according to a distance D between the center of a ball that forms the cornea and the center of the eyeball, as described above. When feature amounts of the eyeballs of a plurality of persons who have the same corneal radius and different iris radiuses are extracted, the feature amounts of the eyeballs have different values according to the iris radiuses.

In the eyeball feature database 123' illustrated in FIG. 24, three feature amounts of the eyeballs having different iris radiuses are registered as a feature amount of the eyeball of a person for which a corneal radius is Rm. A feature amount of the eyeball of a person for which the iris radius is IRm1, a feature amount of the eyeball of a person for which the iris radius is IRm2, and a feature amount of the eyeball of a person for which the iris radius is IRm3 are slightly different from each other. As an example, a feature amount m13 at the time when the person for which the iris radius is IRm1 gazes at a gaze point PG3 is slightly different from a feature amount m23 at the time when the person for which the iris radius is IRm2 gazes at the gaze point PG3. In addition, a feature amount m23 at the time when the person for which the iris radius is IRm2 gazes at the gaze point PG3 is slightly different from a feature amount m33 at the time when the person for which the iris radius is IRm3 gazes at the gaze point PG3. Further, a feature amount m13 at the time when the person for which the iris radius is IRm1 gazes at the gaze point PG3 is slightly different from a feature amount m33 at the time when the person for which the iris radius is IRm3 gazes at the gaze point PG3.

In addition, feature amounts of the eyeballs of a plurality of persons having different corneal radiuses are different from each other, as described above. As an example, a feature amount n3 at the time when a person for which a corneal radius is 4 gazes at the gaze point PG3 has a different value from a feature amount m13, m23 or m33 at the time when a person for which the corneal radius is Rm ($\neq$4) gazes at the gaze point PG3.

Accordingly, in a case in which an eyeball feature of a target person is specified on the basis of only feature amounts of the eyeballs extracted from a plurality of persons having different corneal radiuses, even when the corneal radius is the same or almost the same, a difference in the iris radius generates a difference in the feature amount of the eyeball, and the specified eyeball feature may fail to be an appropriate eyeball feature.

On the other hand, in the eyeball feature database 123' according to this embodiment, a plurality of eyeball features in which the corneal radius is the same and the iris radiuses are different from each other are registered. Accordingly, in a process for determining an eyeball feature, as an example, three eyeball features are first extracted as an eyeball feature for a target person for which the corneal radius is Rm. At this stage, all of the three eyeball features are similar to the eyeball feature of the target person, and may be determined to be the eyeball feature of the target person. However, according to this embodiment, from among the three eyeball features, an eyeball feature in which the iris radius is the same or the closest is determined to be the eyeball feature of the target person. Consequently, an eyeball feature in which the corneal radius and the iris radius are the closest to those of the target person can be determined to be the eyeball feature of the target person, and a calibration parameter can be determined according to a more appropriate eyeball feature.

FIG. 22 illustrates an example of the calibration parameter determination process performed by the line-of-sight detector 1 according to this embodiment. The calibration parameter determination process performed by the line-of-sight detector 1 according to this embodiment may include, for example, a process for correcting the feature amount of the eyeball or the iris diameter according to the orientation of a face, as described in the second embodiment.

In addition, prepared information relating to an eyeball feature including a set of feature amounts of the eyeball at the time of gazing at respective gaze points is not limited to a database, as described above, and the information may be registered (stored) in a storage unit in another mode in which the feature amounts can be discriminated from each other.

Further, the line-of-sight detector 1 according to this embodiment can be applied to various line-of-sight detection systems that detect a line of sight of a target person from an image, similarly to the line-of-sight detector 1 according to the first embodiment.

<Fourth Embodiment>

According to this embodiment, an example is described in which an eyeball feature is approximated to a feature amount of the eyeball of a target person in a process for referring to the eyeball feature database 121 or 123 and determining an eyeball feature of the target person. A functional configuration of a line-of-sight detector 1 according to this embodiment may be, for example, the configuration illustrated in FIG. 1. In addition, an eyeball feature database stored in the line-of-sight detector 1 may be, for example, the eyeball feature database 121 of FIG. 2 or the eyeball feature database 123 of FIG. 20.

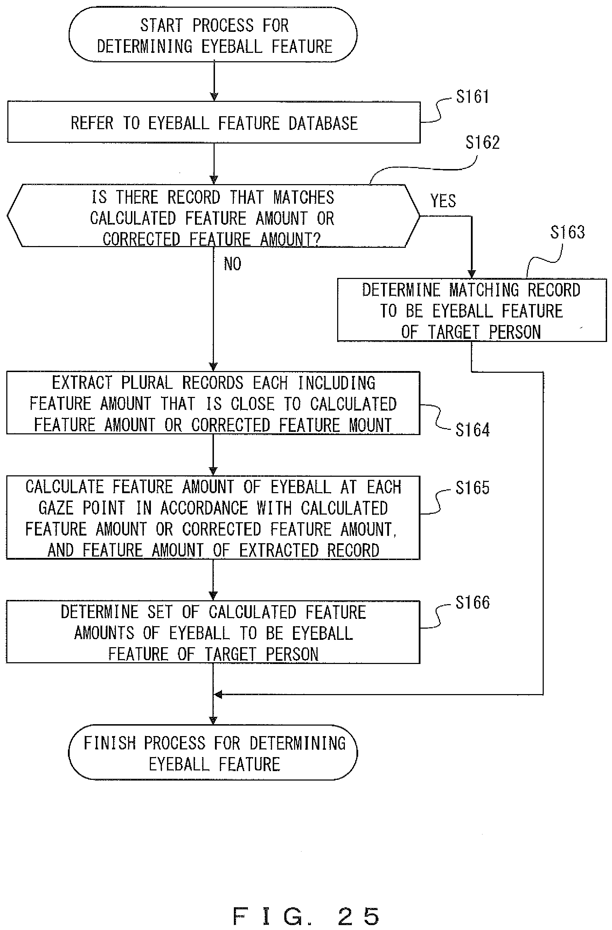

The line-of-sight detector 1 according to this embodiment performs the calibration parameter determination process (step S1) and the line-of-sight detection process (step S2) that are illustrated in FIG. 8. At this time, the line-of-sight detector 1 performs the processing illustrated in FIG. 9 or FIG. 15 as the calibration parameter determination process. The line-of-sight detector 1 performs the processing illustrated in FIG. 25 as the process of step S106 in the calibration parameter determination process, namely, a process for referring to the eyeball feature database and determining an eyeball feature.

FIG. 25 is a flowchart explaining the content of a process for determining an eyeball feature in a calibration parameter determination process according to the fourth embodiment.

A process for determining an eyeball feature (step S106) is performed by the feature determination unit 106 of the line-of-sight detector 1. The feature determination unit 106 according to this embedment first refers to the eyeball feature database 121 (step S161), and determines whether there is a record that matches the calculated feature amount or the corrected feature amount (step S162). The record is a set of a plurality of feature amounts of the eyeball that is associated with one identifier.

When there is a record that matches the calculated feature amount or the corrected feature amount (step S162; YES), the feature determination unit 106 determines the matching record to be an eyeball feature of the target person (step S163). In this case, the feature determination unit 106 transmits the eyeball feature determined in step S163 to the parameter determination unit 107, and finishes the process for determining the eyeball feature.

When there are no records that match the calculated feature amount or the corrected feature amount (step S162; NO), the feature determination unit 106 extracts a plurality of records each including a feature amount that is close to the calculated feature amount or the corrected feature amount (step S164).

The feature determination unit 106 calculates a feature amount of the eyeball at each of the gaze points on the basis of the calculated feature amount or the corrected feature amount, and a feature amount of each of the extracted records (step S165). In step S165, the feature determination unit 106 calculates feature amounts of the eyeball at the respective gaze points according to a ratio of distances between the calculated feature amount or the corrected feature amount, and the respective feature amounts of each of the extracted records.

Then, the feature determination unit 106 determines a set of feature amounts of the eyeball at the respective gaze points that are calculated in step S165 to be an eyeball feature of the target person (step S166). After step S166, the feature determination unit 106 transmits the eyeball feature determined in step S166 to the parameter calculator 107, and finishes the process for determining the eyeball feature.

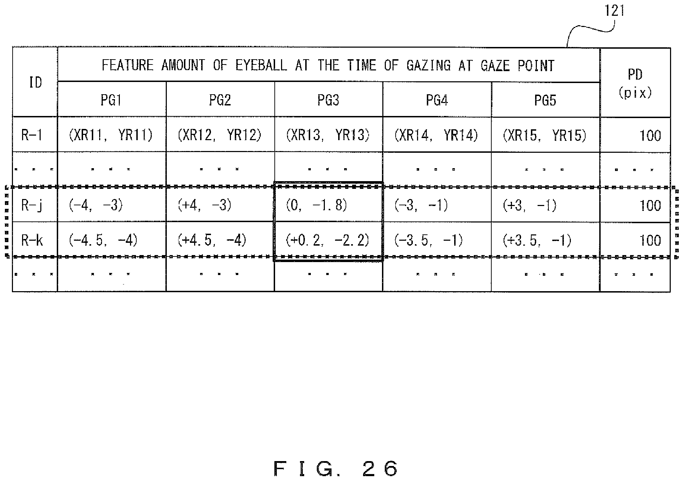

FIG. 26 illustrates examples of a plurality of records extracted from an eyeball feature database. FIG. 26 illustrates examples of records of the right eye that are registered in the eyeball feature database 121.

As an example of the processing above performed by the feature determination unit 106 according to this embodiment, a case is described in which an eyeball feature is determined on the basis of the feature amount (0, -1.9) of the right eye calculated from an image in which a target person is gazing at the gaze point PG3 and the eyeball feature database 121 of FIG. 26. In the eyeball feature database 121 of FIG. 26, a record in which a feature amount at the gaze point PG3 is the feature amount (0, -1.9) has not been registered. Therefore, the feature determination unit 106 determines that there are no records that match the calculated feature amount (step S162; NO), and the feature determination unit 106 extracts a plurality of records that each include a feature amount that is close to the calculated feature amount (0, -1.9) (step S164). In step S164, the feature determination unit 106 extracts two records, a record having the identifier (ID) R-j and a record having the identifier R-k, as a record that includes a feature amount that is close to the calculated feature amount.

The feature determination unit 106 then calculates a feature amount of the eyeball at each of the gaze points on the basis of the calculated feature amount or the corrected feature amount, and the feature amount of the eyeball of each of the extracted records (step S165).

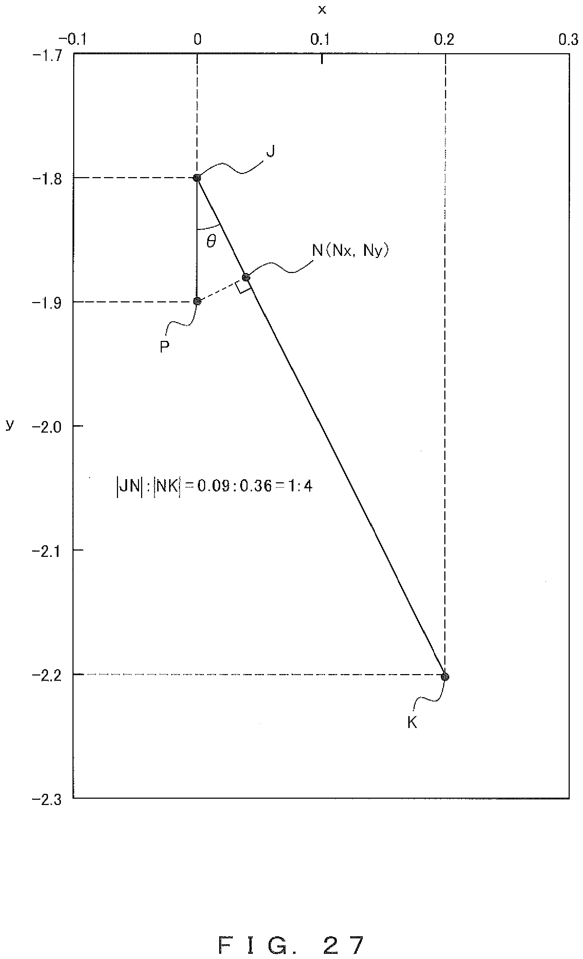

FIG. 27 is a diagram explaining a method for calculating a feature amount.

In calculating a feature amount in step S165, as an example, a calculated feature amount P(0, -1.9) and feature amounts J(0, -1.8) and K(0.2, -2.2) of the extracted records are plotted on a virtual plane on which feature amounts are represented, as illustrated in FIG. 27.

A perpendicular is drawn from the feature amount P to a line segment FK connecting the feature amount J and the feature amount K of the extracted records, and the line segment JK is divided into a line segment JN and a line segment NK at an intersection point N of the line segment JK and the perpendicular. Feature amounts of the eyeball at the time when the target person gazes the respective gaze points are calculated on the basis of a ratio of the divided line segments JN and NK, the record having the identifier R-j, and the record having the identifier R-k.

Assume that an angle that is formed by the line segment JP and the line segment JN is θ. Expression (6-1) and Expression (6-2) below are established.

$$\left|\overrightarrow{JN}\right| = \left|\overrightarrow{JP}\right|\cos\theta \qquad (6-1)$$

$$\overrightarrow{JP}\cdot\overrightarrow{JK} = \left|\overrightarrow{JP}\right|\left|\overrightarrow{JK}\right|\cos\theta \qquad (6-2)$$

In Expression (6-1), JN with an arrow at the top represents a vector from the feature amount J to the intersection point N. In Expression (6-1) and Expression (6-2), JP with an arrow at the top represents a vector from the feature amount J to the feature amount P. In Expression (6-2), JK with an arrow at the top represents a vector from the feature amount J to the feature amount K.

In addition, Expression (6-3) is established from Expression (6-1) and Expression (6-2).

$$\left|\overrightarrow{JN}\right| = \frac{\overrightarrow{JP}\cdot\overrightarrow{JK}}{\left|\overrightarrow{JK}\right|} \qquad (6-3)$$

Accordingly, coordinates of the intersection point N on the virtual plane can be calculated according to Expression (6-4) below.

$$N = J + \left(\frac{\left|\overrightarrow{JN}\right|}{\left|\overrightarrow{JK}\right|}\right)\overrightarrow{JK} \qquad (6-4)$$

When Expression (6-4) is solved by using the feature amount J = (0, -1.8), the vector JN = (Nx, Ny+1.8), and the vector JK = (0.2, -0.4), the coordinates (Nx, Ny) of the intersection point N is (0.04, -1.88). Therefore, a ratio |JN| : |NK| of the line segment JN and the line segment NK is 0.09:0.36 (= 1:4).

After obtaining the ratio of the line segment JN and the line segment NK, the feature determination unit 106 calculates a point at which a line segment connecting a feature amount of the record having the identifier R-j and a feature amount of the record having the identifier R-k into 1:4, for each of the gaze points, and specifies the calculated points to be feature amounts at the time when the target person gazes at the respective gaze points. As an example, feature amounts at the time of gazing at the gaze point PG1 in the records having the identifiers R-j and R-k are (-4, -3) and (-4.5, -4), respectively. Therefore, the feature determination unit 106 calculates a feature amount (X, Y) at the time when the target person gazes at the gaze point PG1 according to Expression (7-1) and Expression (7-2) below.

```
X = -4 + {1 / (1 + 4)} · {-4.5 - (-4)}
  = -4.1                                    (7-1)

Y = -3 + {1 / (1 + 4)} · {-4 - (-3)}
  = -3.2                                    (7-2)
```

As described above, according to this embodiment, in a case in which a feature amount that matches a calculated feature amount or a corrected feature amount has not been registered in an eyeball feature database, an eyeball feature of a target person is determined on the basis of a record including a feature amount that is close to the calculated feature amount or the corrected feature amount. Accordingly, as an example, the number of records registered in the eyeball feature database can be suppressed from increasing, and a processing load of the calibration parameter determination process can be suppressed from increasing due to the enlargement of the database. In addition, according to this embodiment, the eyeball feature of the target person is calculated on the basis of a distance from the calculated feature amount or the corrected feature amount to a feature amount of the extracted record. Accordingly, as an example, a feature amount that is closer to an actual eyeball feature of the target person can be calculated in comparison with a case in which a record including a feature amount that is the closest to the calculated feature amount or the corrected feature amount to be the eyeball feature of the target person, and the accuracy of detection of a line of sight is enhanced.

The flowchart of FIG. 25 is an example of a process for determining an eyeball feature in the calibration parameter determination process. The process for determining the eyeball feature can be changed appropriately, and is not limited to the procedure illustrated in FIG. 25, and the content of some processes may be changed.

In addition, prepared information relating to the eyeball feature including a set of feature amounts of the eyeball at the time of gazing at respective gaze points is not limited to a database, as described above, and the information may be registered (stored) in a storage unit in another mode in which the feature amounts can be discriminated from each other.

Further, the line-of-sight detector 1 according to this embodiment can be applied to various line-of-sight detection systems that detect a line of sight of a target person from an image, similarly to the line-of-sight detector 1 according to the first embodiment.

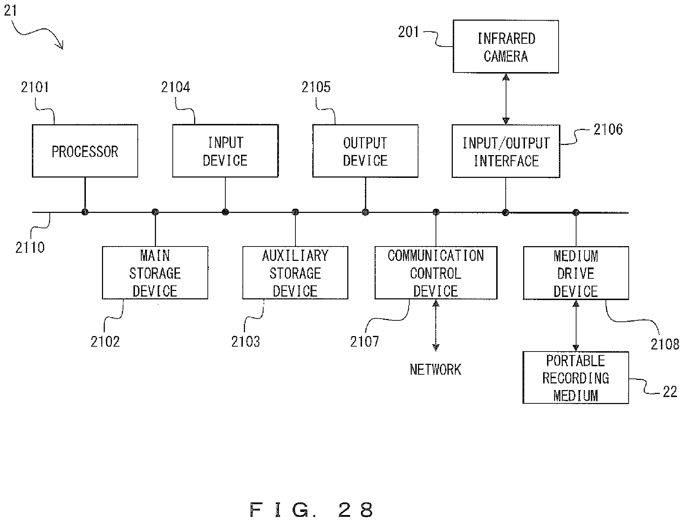

The line-of-sight detector 1 according to each of the embodiments above can be realized by using, for example, a computer and a program executed by the computer. A line-of-sight detector 1 that is realized by using a computer and a program is described below with reference to FIG. 28.

FIG. 28 illustrates a hardware configuration of a computer.

As illustrated in FIG. 28, a computer 21 includes a processor 2101, a main storage device 2102, an auxiliary storage device 2103, an input device 2104, an output device 2105, an input/output interface 2106, a communication control device 2107, and a medium drive device 2108. These components 2101 to 2108 in the computer 21 are mutually connected via a bus 2110, and data can be communicated among these components.

The processor 2101 is a central processing unit (CPU), a micro processing unit (MPU), or the like. The processor 2101 controls the entire operation of the computer 21 by executing various programs including an operating system. The processor 2101 also performs, for example, the calibration parameter determination process and the line-of-sight detection process illustrated in FIGS. 8 to 10.

The main storage device 2102 includes a read only memory (ROM) and a random access memory (RAM) that are not illustrated. In the ROM of the main storage device 2102, as an example, a prescribed basic control program that the processor 2101 reads at the time of starting the computer 21 has been registered. The processor 2101 uses the RAM of the main storage device 2102 as a working storage area as needed at the time of executing various programs. The RAM of the main storage device 2102 can be used, for example, to store an image obtained from the infrared camera 201, a feature amount of the eyeball calculated according to the image, a calibration parameter, and the like.

The auxiliary storage device 2103 is, for example, a non-volatile memory (including a solid state drive (SSD)), such as a flash memory, or a hard disk drive (HDD). The auxiliary storage device 2103 can be used to store the various programs executed by the processor 2101, various types of data, and the like. The auxiliary storage device 2103 can be used to store, for example, a program including the calibration parameter determination process and the line-of-sight detection process. In addition, the auxiliary storage device 2103 can be used, for example, to store the eyeball feature database 121 or 123, an image obtained from the infrared camera 201, a calibration parameter, and the like, or to store a history of a detected line of sight or the like.

The input device 2104 is, for example, a keyboard device or a touch panel device. When an operator (a user) of the computer 21 performs a prescribed operation on the input device 2104, the input device 2104 transmits input information associated with the content of the operation to the processor 2101. The input device 2104 can be used, for example, as the input devices 3 of FIGS. 1 and 19.

The output device 2105 is, for example, a display device or a speaker. The output device 2105 can be used, for example, to report a gaze point to be gazed at to a target person. Namely, the output device 2105 can be used as the output devices 4 of FIGS. 1 and 19.

The input/output interface 2106 connects the computer 21 and another electronic device. The input/output interface 2106 includes, for example, a connector of the universal serial bus (USB) standard. The input/output interface 2106 can be used, for example, to connect the computer 21 and the infrared camera 201 of the line-of-sight sensor 2.

The communication control device 2107 is a device that connects the computer 21 to a network, and that controls various communication between the computer 21 and another electronic device via the network. The communication control device 2107 can be used, for example, to obtain the eyeball feature database 121 or 123 generated by the database generator 7 and store the eyeball feature database 121 or 123 in the auxiliary storage device 2103 or the like. The communication control device 2107 can also be used to perform communication between the computer 21 (the line-of-sight detector 1) and the management server 15, as in the line-of-sight detection system 17 illustrated in FIG. 14A.

The medium drive device 2108 reads a program and data registered in a portable storage medium 22, and writes data or the like that is stored in the auxiliary storage device 2103 to the portable storage medium 22. A reader/writer for a memory card that conforms to one or more types of standards can be used, for example, as the medium drive device 2108. In a case in which the reader/writer for the memory card is used as the medium drive device 2108, a memory card (a flash memory) of a standard that the reader/writer for the memory card conforms to, for example, the secure digital (SD) standard, can be used, for example, as the portable storage medium 22. In addition, a flash memory including a connector of the USB standard can be used, for example, as the portable recording medium 22. The portable recording medium 22 can be used to store a program including the calibration parameter determination process and the line-of-sight detection process above, the eyeball feature database 121 or 123, a calibration parameter, a detected line of sight, and the like.

In a case in which the computer 21 is mounted with an optical disk drive that can be used as the medium drive device 2108, various types of optical disks that can be recognized by the optical disk drive can be used as the portable recording medium 22. Examples of an optical disk that can be used as the portable recording medium 22 include a compact disc (CD), a digital versatile disc (DVD), and a Blu-ray disc (Blu-ray is a registered trademark).

When an instruction to start the processing of FIG. 8 is input, the computer 21 reads and executes a line-of-sight detection program including the calibration parameter determination process and the line-of-sight detection process from a non-transitory recording medium such as the auxiliary storage device 2103. At this time, the processor 2101 functions (operates) as the image obtaining unit 101, a gaze point instruction unit 102, the feature amount calculator 103, the feature amount corrector 105, the feature determination unit 106, the parameter calculator 107, and the line-of-sight calculator 108. Further, in the case of a line-of-sight detection program for causing the computer 21 to execute the processing described in the third embodiment, the processor 2101 also functions (operates) as the iris radius calculator 110. Furthermore, the RAM of the main storage device 2102, the auxiliary storage device 2103, and the like function as a storage unit that stores the eyeball feature database 121 or 123, and the line-of-sight storage unit 122 that stores a detected line of sight.

Further, the computer 21 is not made to operate only as the line-of-sight detector 1, but can also be made to operate as the database generator 7. In a case in which the computer 21 is made to operate as the database generator 7, the processor 2101 is made to execute a program including the generation process of FIG. 5. In this case, the processor 2101 functions (operates) as the gazing instruction unit 701, the image obtaining unit 702, the feature amount calculator 703, the distance calculator 704, and the registration unit 705 that are illustrated in FIG. 4. In addition, the RAM of the main storage device 2102, the auxiliary storage device 2103, and the like function as a storage unit that stores information indicating a plurality of gaze points, a storage unit that stores an image obtained from the infrared camera 201, and a storage unit that stores the eyeball feature database 121.

The computer 21 that is made to operate as the line-of-sight detector 1 or the database generator 7 does not need to include all of the components 2101 to 2108 illustrated in FIG. 28, and some of the components can be omitted according to applications or conditions. As an example, the communication control device 2107 or the medium drive device 2108 may be omitted from the computer 21.

**Claims**

1. A line-of-sight detector (1) comprising:

   a storage unit (121) in which a plurality of eyeball features are registered in a mode of associating each of the plurality of eyeball features with a corneal radius, each of the plurality of eyeball features including a set of feature amounts of an eyeball that are detected when respective gaze points that have been specified are gazed at from a point of view that has a prescribed position relationship with a camera;
   a feature amount calculator (103) configured to calculate a feature amount of an eyeball of a person who is gazing at a first gaze point of the gaze points in accordance with an image captured by the camera (201) that has a prescribed position relationship with the person;
   a feature determination unit (106) configured to determine an eyeball feature of the person in accordance with a feature amount of the eyeball that corresponds to the first gaze point in the plurality of eyeball features registered in the storage unit, and the calculated feature amount of the eyeball of the person; and
   a parameter calculator (108) configured to calculate a calibration parameter for a line of sight of the person in accordance with the feature amounts of the eyeball that are included in the determined eyeball feature.

2. The line-of-sight detector according to claim 1, wherein
   the feature amount of the eyeball is a value that represents a position relationship between a center of a pupil and a center of a corneal reflex of the eyeball of the person that is photographed in the image.

3. The line-of-sight detector according to claim 2, wherein

each of the plurality of eyeball features registered in a memory further includes information indicating a distance from the camera to the point of view, and
the line-of-sight detector further includes:

a feature corrector (105) configured to calculate a distance from the camera to the person in accordance with the image, and to correct the feature amount of the eyeball calculated by the feature amount calculator in accordance with the calculated distance and the information indicating the distance, the information being registered in the storage unit.

4. The line-of-sight detector according to claim 3, wherein
the information indicating the distance from the camera to the point of view, and the distance calculated by the feature amount corrector are distances of the pupils and distances of the corneal reflexes of both eyes of the person in the image.

5. The line-of-sight detector according to claim 1, wherein
the feature amount of the eyeball is a value that represents a position relationship between a center of a pupil and a center of a corneal reflex of the eyeball of the person photographed in the image, and
the line-of-sight detector further includes a feature amount corrector (105) configured to correct the feature amount of the eyeball calculated by the feature amount calculator in accordance with a difference between the feature amount of the eyeball of a right eye photographed in an obtained image and the feature amount of the eyeball of a left eye.

6. The line-of-sight detector according to claim 5, wherein
the feature amounts of the eyeball registered in a memory are feature amounts at a time of gazing at the respective gaze points in a state in which a face is facing the camera, and
when the difference between the feature amount of the eyeball of the right eye and the feature amount of the eyeball of the left eye is greater than or equal to a threshold, the feature amount corrector corrects one of the feature amount of the eyeball of the right eye and the feature amount of the eyeball of the left eye so as to match another of the feature amount of the eyeball of the right eye and the feature amount of the eyeball of the left eye.

7. The line-of-sight detector according to claim 1, wherein
each of the plurality of eyeball features registered in a memory includes an iris radius of the eyeball,
the line-of-sight detector further includes an iris radius calculator (109) configured to calculate the iris radius of the eyeball of the person in accordance with an obtained image, and
the feature determination unit (106) determines the eyeball feature of the person in accordance with the feature amount of the eyeball that corresponds to the first gaze point, and the iris radius.

8. The line-of-sight detector according to claim 1, wherein
the feature determination unit determines an eyeball feature in which the feature amount of the eyeball that corresponds to the first gaze point matches the calculated feature amount of the eyeball of the person, or an eyeball feature in which the feature amount of the eyeball that corresponds to the first gaze point is the closest to the calculated feature amount of the eyeball of the person, from among the plurality of eyeball features registered in a memory, to be the eyeball feature of the person.

9. The line-of-sight detector according to claim 1, wherein
the feature determination unit performs:

extracting, from among the plurality of eyeball features registered in a memory, two eyeball features in which the feature amount of the eyeball that corresponds to the first gaze point is close to the calculated feature amount of the eyeball of the person;
estimating feature amounts of the eyeball at a time when the person gazes at the respective gaze points in accordance with a distance between the feature amount of the eyeball in one eyeball feature of the extracted eyeball features and the calculated feature amount of the eyeball, and a distance between the feature amount of the eyeball in another eyeball feature of the extracted eyeball features and the calculated feature amount of the eyeball; and
determining a set of the estimated feature amounts of the eyeball to be the eyeball feature of the person.

10. A line-of-sight detection method for detecting a line of sight of a person that is photographed in an image captured by a camera (201), the line-of-sight detection method comprising:

calculating (S102), by a computer (21), a feature amount of an eyeball of a person who is gazing at a first gaze point of gaze points that have been specified in accordance with the image of the person, the image being obtained from the camera;

referring, by the computer, to registration information (121) including a plurality of eyeball features that are registered in a memory in a mode of associating each of the plurality of eyeball features with a corneal radius, each of the plurality of eyeball features including a set of feature amounts of the eyeball that are detected when the respective gaze points are gazed at from a point of view that has a prescribed position relationship with the camera, and determining (S106), by the computer, the eyeball feature of the person in accordance with the feature amount of the eyeball that corresponds to the first gaze point in the plurality of the eyeball features and the calculated feature amount of the eyeball of the person; and

calculating (S107), by the computer, a calibration parameter for the line of sight of the person in accordance with the feature amounts of the eyeball that are included in the determined eyeball feature.

11. The line-of-sight detection method according to claim 10, each of the plurality of eyeball features in the registration information including an interpupillary distance between a pupil of a right eye and a pupil of a left eye, the line-of-sight detection method further comprising:

after the calculating the feature amount of the eyeball of the person, correcting (S105), by the computer, the calculated feature amount of the eyeball of the person in accordance with an interpupillary distance of the person that is calculated from the image and the interpupillary distance of the eyeball feature, wherein

when the feature amount of the eyeball is corrected, the computer determines the eyeball feature of the person in accordance with the corrected feature amount of the eyeball and the registration information in the determining the eyeball feature of the person.

12. The line-of-sight detection method according to claim 10, wherein
the determining the eyeball feature of the person includes:

extracting (S164), by the computer, two eyeball features in which the feature amount of the eyeball that corresponds to the first gaze point is close to the calculated feature amount of the eyeball of the person, from among the plurality of eyeball features in the registration information;

estimating (S165), by the computer, feature amounts of the eyeball at a time when the person gazes at the respective gaze points in accordance with a distance between the feature amount of the eyeball in one eyeball feature of the extracted eyeball features and the calculated feature amount of the eyeball, and a distance between the feature amount of the eyeball in another eyeball feature of the extracted eyeball features and the calculated feature amount of the eyeball; and

determining (S166), by the computer, a set of the estimated feature amounts of the eyeball to be the eyeball feature of the person.

13. A line-of-sight detection program that causes a computer (21) to execute a process comprising:

calculating (S102) a feature amount of an eyeball of a person who is gazing at a first gaze point of gaze points that have been specified in accordance with an image of the person, the image being obtained from a camera(201);

referring to registration information including a plurality of eyeball features that are registered in a memory in a mode of associating each of the plurality of eyeball features with a corneal radius, each of the plurality of eyeball features including a set of feature amounts of the eyeball that are detected when the respective gaze points are gazed at from a point of view that has a prescribed position relationship with the camera, and determining (S106) the eyeball feature of the person in accordance with the feature amount of the eyeball that corresponds to the first gaze point in the plurality of the eyeball features and the calculated feature amount of the eyeball of the person; and

calculating (S107) a calibration parameter for a line of sight of the person in accordance with the feature amounts of the eyeball that are included in the determined eyeball feature.

LINE-OF-SIGHT DETECTOR

```
 4 ─┐   ┌──────────┐                    ┌──────────────┐
    └─  │ OUTPUT   │◄─────────┐         │   GAZING     │
        │ DEVICE   │          └─────────│ INSTRUCTION  │─── 102
        └──────────┘                    │    UNIT      │
                                        └──────────────┘
                                               ▲
 2 ─┐  ┌──────────────┐                 ┌──────────────┐─── 101
    └─ │              │                 │    IMAGE     │
       │ ┌──────────┐ │────────────────►│  OBTAINING   │
 201 ──┼─│ INFRARED │ │         ┌──────►│    UNIT      │
       │ │ CAMERA   │ │         │       └──────────────┘
       │ └──────────┘ │         │              │
       │              │         │       ┌──────────────┐─── 103
       │ ┌──────────┐ │         │       │   FEATURE    │
 202 ──┼─│ INFRARED │ │         │       │   AMOUNT     │
       │ │   LED    │ │         │       │  CALCULATOR  │
       │ └──────────┘ │         │       └──────────────┘
       └──────────────┘         │              │
                                │       ┌────────────┐    ┌──────────────┐
 3 ─┐  ┌──────────────┐         │   105 │  PROCESS   │    │   EYEBALL    │─── 121
    └─ │    INPUT     │         │       │ SWITCHING  │    │   FEATURE    │
       │    DEVICE    │─────────●──────►│   UNIT     │    │  DATABASE    │
       └──────────────┘                 └────────────┘    └──────────────┘
                                          │    ▲ │                │
                                 ┌────────┴─┐  │ │  ┌──────────┐  │
                           104   │ FEATURE  │◄─┘ │  │ FEATURE  │◄─┘─── 106
                                 │ AMOUNT   │────┼─►│DETERMINA-│
                                 │CORRECTOR │    │  │TION UNIT │
                                 └──────────┘    │  └──────────┘
                                                 │       │
                                                 │  ┌──────────┐─── 107
                                                 │  │PARAMETER │
                                                 └──│CALCULATOR│
                                                    └──────────┘
                                                          │
 1 ─┐                          108 ─┐  ┌──────────┐       │  ┌──────────┐─── 122
    └─                             └─ │ LINE-OF- │◄───────┘  │ LINE-OF- │
                                      │  SIGHT   │──────────►│  SIGHT   │
                                      │CALCULATOR│           │STORAGE   │
                                      └──────────┘           │  UNIT    │
                                                             └──────────┘
```

F I G.  1

121

| ID | FEATURE AMOUNT OF EYEBALL AT THE TIME OF GAZING AT GAZE POINT | | | | | PD (pix) |
|---|---|---|---|---|---|---|
| | PG1 | PG2 | PG3 | PG4 | PG5 | |
| RIGHT EYE | | | | | | |
| R-1 | (XR11, YR11) | (XR12, YR12) | (XR13, YR13) | (XR14, YR14) | (XR15, YR15) | 100 |
| ... | ... | ... | ... | ... | ... | ... |
| R-k | (-5, -7) | (+5, -7) | (0, -4) | (-4, -2) | (+4, -2) | 100 |
| ... | ... | ... | ... | ... | ... | ... |
| LEFT EYE | | | | | | |
| L-1 | (XL11, YL11) | (XL12, YL12) | (XL13, YL13) | (XL14, YL14) | (XL15, YL15) | 100 |
| ... | ... | ... | ... | ... | ... | ... |
| L-k | (XLk1, YLk1) | (XLk2, YLk2) | (XLk3, YLk3) | (XLk4, YLk4) | (XLk5, YLk5) | 100 |
| ... | ... | ... | ... | ... | ... | ... |

F I G. 2

F I G. 3 A

F I G.  3 B

3 — INPUT DEVICE

DATABASE GENERATOR

4 — OUTPUT DEVICE

GAZING INSTRUCTION UNIT — 701

2 —

201 — INFRARED CAMERA

202 — INFRARED LED

IMAGE OBTAINING UNIT — 702

FEATURE AMOUNT CALCULATOR

703

DISTANCE CALCULATOR — 704

REGISTRATION UNIT — 705

121 — EYEBALL FEATURE DATABASE

7 —

F I G. 4

START

SPECIFY ONE GAZE POINT OF GAZE POINTS — S11

OBTAIN IMAGE INCLUDING EYEBALL OF PERSON
WHO IS GAZING AT SPECIFIED GAZE POINT — S12

HAVE ALL GAZE POINTS BEEN SPECIFIED? — S13

NO

YES

EXTRACT PUPIL AND CORNEAL REFLEX
FROM EACH IMAGE, AND
CALCULATE FEATURE AMOUNT OF EYEBALL — S14

CALCULATE INTERPUPILLARY DISTANCE
ACCORDING TO POSITION OF
PUPIL EXTRACTED FROM EACH IMAGE — S15

REGISTER FEATURE AMOUNT OF EYEBALL
AND INTERPUPILLARY DISTANCE — S16

WILL GENERATION PROCESS BE CONTINUED? — S17

YES

NO

END

F I G.   5

FRONT SIDE
OF VEHICLE

REAR SIDE
OF VEHICLE

F I G. 6 A

FRONT SIDE
OF VEHICLE

REAR SIDE
OF VEHICLE

2        801            8

9

1        4        3

803

F I G.  6 B

F I G. 7 A

F I G. 7 B

START

CALIBRATION PARAMETER DETERMINATION PROCESS — S1

LINE-OF-SIGHT DETECTION PROCESS — S2

END

F I G. 8

```
        ╭─────────────────────────────╮
        │   START CALIBRATION PARAMETER │
        │    DETERMINATION PROCESS      │
        ╰─────────────────────────────╯
                       │
                       ▼
   ┌─────────────────────────────────────────┐
   │ OBTAIN IMAGE INCLUDING EYEBALL OF TARGET │ ─── S101
   │ PERSON THAT IS CAPTURED IN STATE IN WHICH│
   │ TARGET PERSON IS GAZING AT ONE GAZE POINT│
   └─────────────────────────────────────────┘
                       │
                       ▼
   ┌─────────────────────────────────────────┐
   │    EXTRACT PUPIL AND CORNEAL REFLEX      │ ─── S102
   │        FROM OBTAINED IMAGE, AND          │
   │    CALCULATE FEATURE AMOUNT OF EYEBALL   │
   └─────────────────────────────────────────┘
                       │
                       ▼
   ┌─────────────────────────────────────────┐
   │ CALCULATE INTERPUPILLARY DISTANCE ACCORDING │ ─── S103
   │ TO POSITION OF PUPIL EXTRACTED FROM IMAGE │
   └─────────────────────────────────────────┘
                       │                          S104
                       ▼
        ╱───────────────────────────────╲
       ╱    DOES CALCULATED INTERPUPILLARY ╲  YES
      ╱  DISTANCE MATCH INTERPUPILLARY DISTANCE ╲──────┐
       ╲    IN EYEBALL FEATURE DATABASE?    ╱          │
        ╲───────────────────────────────╱             │
                       │ NO                            │
                       ▼                               │
   ┌─────────────────────────────────────────┐        │
   │ CORRECT FEATURE AMOUNT OF EYEBALL ACCORDING│ ─ S105 │
   │   TO RATIO OF INTERPUPILLARY DISTANCES    │        │
   └─────────────────────────────────────────┘        │
                       │◀──────────────────────────────┘
                       ▼
   ┌─────────────────────────────────────────┐
   │     REFER TO EYEBALL FEATURE DATABASE,    │
   │      AND DETERMINE EYEBALL FEATURE        │
   │         USED FOR CALIBRATION              │ ─── S106
   └─────────────────────────────────────────┘
                       │
                       ▼
   ┌─────────────────────────────────────────┐
   │     CALCULATE CALIBRATION PARAMETER       │ ─── S107
   │      ACCORDING TO EYEBALL FEATURE         │
   └─────────────────────────────────────────┘
                       │
                       ▼
        ╭─────────────────────────────╮
        │  FINISH CALIBRATION PARAMETER │
        │    DETERMINATION PROCESS      │
        ╰─────────────────────────────╯
```

F I G. 9

START LINE-OF-SIGHT
DETECTION PROCESS

OBTAIN IMAGE INCLUDING EYEBALL
OF TARGET PERSON — S201

EXTRACT PUPIL AND CORNEAL REFLEX
FROM OBTAINED IMAGE, AND
CALCULATE FEATURE AMOUNT OF EYEBALL — S202

DETECT LINE OF SIGHT OF TARGET PERSON
ACCORDING TO CALCULATED FEATURE AMOUNT
OF EYEBALL AND CALIBRATION PARAMETER — S203

STORE DETECTED LINE OF SIGHT
IN LINE-OF-SIGHT STORAGE UNIT — S204

S205

YES

WILL DETECTION PROCESS BE CONTINUED?

NO

FINISH LINE-OF-SIGHT
DETECTION PROCESS

F I G. 1 0

F I G. 1 1

F I G. 1 2

14

13

1201

12

PG3

PG1

PG2

INFORMATION
PROCESSING
DEVICE

PG5

9

9S

LINE-OF-
SIGHT
DETECTOR

202

1

201

PG4

F I G. 1 3

17

| LINE-OF-<br>SIGHT<br>DETECTOR | | MANAGEMENT<br>SERVER |
| --- | --- | --- |
| | | EYEBALL<br>FEATURE<br>DATABASE |

1

16

121          15

F I G.   1 4 A

MANAGEMENT SERVER — 15

121 — EYEBALL FEATURE DATABASE

LINE-OF-SIGHT HISTORY — 1501

17

16

804

8(8A)

VEHICLE

1 — LINE-OF-SIGHT DETECTOR

804

VEHICLE — 8(8B)

LINE-OF-SIGHT DETECTOR

. . . .

— 1

F I G. 1 4 B

START CALIBRATION PARAMETER
DETERMINATION PROCESS

OBTAIN IMAGE INCLUDING EYEBALL OF TARGET
PERSON THAT IS CAPTURED IN STATE IN WHICH
TARGET PERSON IS GAZING AT ONE GAZE POINT ⟋ S101

EXTRACT PUPIL AND CORNEAL REFLEX
FROM OBTAINED IMAGE,
AND CALCULATE FEATURE AMOUNT OF EYEBALL ⟋ S102

CORRECT FEATURE AMOUNT OF EYEBALL ⟋ S110

REFER TO EYEBALL FEATURE DATABASE,
AND DETERMINE EYEBALL FEATURE USED
FOR CALIBRATION ⟋ S106

CALCULATE CALIBRATION PARAMETER
ACCORDING TO EYEBALL FEATURE ⟋ S107

FINISH CALIBRATION PARAMETER
DETERMINATION PROCESS

F I G. 1 5

```
            ┌─────────────────────────────────┐
            │   START PROCESS FOR CORRECTING   │
            │    FEATURE AMOUNT OF EYEBALL     │
            └─────────────────────────────────┘
                             │
                             ▼
        ┌──────────────────────────────────────────┐ ⌐ S111
        │ ESTIMATE ORIENTATION OF FACE ACCORDING TO │
        │        FEATURE AMOUNT OF EYEBALL          │
        └──────────────────────────────────────────┘
                             │                      S112
                             ▼
        ╱─────────────────────────────────────╲ YES
        ╲      IS FACE FACING CAMERA?          ╱──────┐
         ╲───────────────────────────────────╱        │
                             │ NO                      │
                             ▼                         │
        ┌──────────────────────────────────────┐ ⌐ S113│
        │  CORRECT FEATURE AMOUNT OF EYEBALL    │      │
        │  OF RIGHT EYE OR LEFT EYE ACCORDING   │      │
        │         TO ORIENTATION OF FACE        │      │
        └──────────────────────────────────────┘      │
                             │◄──────────────────────────┘
                             ▼
        ┌──────────────────────────────────────┐
        │     CALCULATE INTERPUPILLARY DISTANCE │
        └──────────────────────────────────────┘
                             │              ⌐ S114
                             ▼
        ╱─────────────────────────────────────╲ YES
        ╱   DOES CALCULATED INTERPUPILLARY      ╲─────┐
        ╲ DISTANCE MATCH INTERPUPILLARY DISTANCE╱      │
         ╲   IN EYEBALL FEATURE DATABASE?     ╱        │
                             │ NO        S104          │
                             ▼                 S105    │
        ┌──────────────────────────────────────┐ ⌐    │
        │ CORRECT FEATURE AMOUNT OF EYEBALL ACCORDING  │
        │   TO RATIO OF INTERPUPILLARY DISTANCES │     │
        └──────────────────────────────────────┘      │
                             │◄──────────────────────────┘
                             ▼
            ┌─────────────────────────────────┐
            │   FINISH PROCESS FOR CORRECTING  │
            │     FEATURE AMOUNT OF EYEBALL    │
            └─────────────────────────────────┘
```

F I G.   1 6

F I G. 1 7

F I G.  1 8

4 — OUTPUT DEVICE

LINE-OF-SIGHT DETECTOR

GAZING INSTRUCTION UNIT — 102

2 — 

201 — INFRARED CAMERA

202 — INFRARED LED

IMAGE OBTAINING UNIT — 101

EYEBALL FEATURE DATABASE — 123

FEATURE AMOUNT CALCULATOR — 103

FEATURE AMOUNT CORRECTOR

FEATURE DETERMINATION UNIT — 106

105

3 — INPUT DEVICE

PROCESS SWITCHING UNIT — 104

IRIS RADIUS CALCULATOR

109

PARAMETER CALCULATOR — 107

1 — 

108 — LINE-OF-SIGHT CALCULATOR

LINE-OF-SIGHT STORAGE UNIT — 122

F I G.   1 9

123

| | ID | IR | FEATURE AMOUNT OF EYEBALL AT THE TIME OF GAZING AT GAZE POINT | | | | | PD (pix) |
|---|---|---|---|---|---|---|---|---|
| | | | PG1 | PG2 | PG3 | PG4 | PG5 | |
| RIGHT EYE | R-1 | IR1 | (XR11, YR11) | (XR12, YR12) | (XR13, YR13) | (XR14, YR14) | (XR15, YR15) | 100 |
| | . . . | . . . | . . . | . . . | . . . | . . . | . . . | . . . |
| | R-k | 8 | (-5, -7) | (+5, -7) | (0, -4) | (-4, -2) | (+4, -2) | 100 |
| | . . . | . . . | . . . | . . . | . . . | . . . | . . . | . . . |
| LEFT EYE | L-1 | IL1 | (XL11, YL11) | (XL12, YL12) | (XL13, YL13) | (XL14, YL14) | (XL15, YL15) | 100 |
| | . . . | . . . | . . . | . . . | . . . | . . . | . . . | . . . |
| | L-k | ILk | (XLk1, YLk1) | (XLk2, YLk2) | (XLk3, YLk3) | (XLk4, YLk4) | (XLk5, YLk5) | 100 |
| | . . . | . . . | . . . | . . . | . . . | . . . | . . . | . . . |

F I G. 2 0

F I G. 2 1

START CALIBRATION PARAMETER
DETERMINATION PROCESS

OBTAIN IMAGE INCLUDING EYEBALL OF TARGET
PERSON THAT IS CAPTURED IN STATE IN WHICH
TARGET PERSON IS GAZING AT ONE GAZE POINT    S101

EXTRACT PUPIL AND CORNEAL REFLEX
FROM OBTAINED IMAGE, AND
CALCULATE FEATURE AMOUNT OF EYEBALL    S102

EXTRACT IRIS RADIUS FROM OBTAINED IMAGE    S120

CALCULATE INTERPUPILLARY DISTANCE ACCORDING
TO POSITION OF PUPIL EXTRACTED FROM IMAGE    S103

S104

DOES CALCULATED INTERPUPILLARY
DISTANCE MATCH INTERPUPILLARY DISTANCE
IN EYEBALL FEATURE DATABASE?    YES

NO

CORRECT FEATURE AMOUNT OF EYEBALL
AND IRIS RADIUS ACCORDING TO
RATIO OF INTERPUPILLARY DISTANCES    S121

REFER TO EYEBALL FEATURE DATABASE,
AND DETERMINE EYEBALL FEATURE
USED FOR CALIBRATION    S106

CALCULATE CALIBRATION PARAMETER
ACCORDING TO EYEBALL FEATURE    S107

FINISH CALIBRATION PARAMETER
DETERMINATION PROCESS

F I G.  2 2

FIG. 23

123'

| CR | IR | PG1 | PG2 | PG3 | PG4 | PG5 |
|----|----|----|----|----|----|----|
| 4 | IR1 | n1 | n2 | n3 | n4 | n5 |
| . . . | . . . | . . . | . . . | . . . | . . . | . . . |
| Rm | IRm1 | m11 | m12 | m13 | m14 | m15 |
| Rm | IRm2 | m21 | m22 | m23 | m24 | m25 |
| Rm | IRm3 | m31 | m32 | m33 | m34 | m35 |
| . . . | . . . | . . . | . . . | . . . | . . . | . . . |
| 9 | IRk | k1 | k2 | k3 | k4 | k5 |
| . . . | . . . | . . . | . . . | . . . | . . . | . . . |

F I G.  2 4

START PROCESS FOR
DETERMINING EYEBALL FEATURE

REFER TO EYEBALL FEATURE DATABASE — S161

IS THERE RECORD THAT MATCHES
CALCULATED FEATURE AMOUNT OR
CORRECTED FEATURE AMOUNT? — S162

YES

NO

DETERMINE MATCHING RECORD
TO BE EYEBALL FEATURE
OF TARGET PERSON — S163

EXTRACT PLURAL RECORDS EACH INCLUDING
FEATURE AMOUNT THAT IS CLOSE TO CALCULATED
FEATURE AMOUNT OR CORRECTED FEATURE MOUNT — S164

CALCULATE FEATURE AMOUNT OF EYEBALL AT EACH
GAZE POINT IN ACCORDANCE WITH CALCULATED
FEATURE AMOUNT OR CORRECTED FEATURE AMOUNT,
AND FEATURE AMOUNT OF EXTRACTED RECORD — S165

DETERMINE SET OF CALCULATED FEATURE
AMOUNTS OF EYEBALL TO BE EYEBALL
FEATURE OF TARGET PERSON — S166

FINISH PROCESS FOR DETERMINING
EYEBALL FEATURE

F I G. 2 5

121

| ID | FEATURE AMOUNT OF EYEBALL AT THE TIME OF GAZING AT GAZE POINT | | | | | PD (pix) |
| | PG1 | PG2 | PG3 | PG4 | PG5 | |
|---|---|---|---|---|---|---|
| R-1 | (XR11, YR11) | (XR12, YR12) | (XR13, YR13) | (XR14, YR14) | (XR15, YR15) | 100 |
| . . . | . . . | . . . | . . . | . . . | . . . | . . . |
| R-j | (−4, −3) | (+4, −3) | (0, −1.8) | (−3, −1) | (+3, −1) | 100 |
| R-k | (−4.5, −4) | (+4.5, −4) | (+0.2, −2.2) | (−3.5, −1) | (+3.5, −1) | 100 |
| . . . | . . . | . . . | . . . | . . . | . . . | . . . |

F I G. 2 6

F I G. 2 7

21

| | | | 201 — INFRARED CAMERA |

2101  2104  2105

| PROCESSOR | INPUT DEVICE | OUTPUT DEVICE | INPUT/OUTPUT INTERFACE — 2106 |

2110

| MAIN STORAGE DEVICE | AUXILIARY STORAGE DEVICE | COMMUNICATION CONTROL DEVICE | MEDIUM DRIVE DEVICE |

2102  2103  2107  2108

NETWORK

PORTABLE RECORDING MEDIUM — 22

F I G.  2 8

Europäisches
Patentamt

European
Patent Office

Office européen
des brevets

# EUROPEAN SEARCH REPORT

Application Number

EP 17 16 7719

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | JP H07 35966 A (NIPPON KOGAKU KK)<br>7 February 1995 (1995-02-07)<br>* abstract *<br>* paragraphs [0001], [0009], [0010], [0014] *<br>* paragraph [0042] - paragraph [0044] *<br>* figures 1-11 * | 1,2,5,<br>10,13 | INV.<br>A61B3/113<br>A61B3/15 |
| X | JP 2015 144635 A (JVC KENWOOD CORP)<br>13 August 2015 (2015-08-13) | 1 | |
| Y | * abstract *<br>* paragraph [0015] - paragraph [0050] * | 3,4,6-9,<br>11,12 | |
| X | WO 2015/146491 A1 (JVC KENWOOD CORP [JP])<br>1 October 2015 (2015-10-01) | 1 | |
| Y | * paragraph [0010] - paragraph [0096] *<br>* figures 1-16 * | 3,4,6-9,<br>11,12 | |
| A | US 2007/279590 A1 (EBISAWA YOSHINOBU [JP])<br>6 December 2007 (2007-12-06)<br>* abstract *<br>* paragraph [0016] - paragraph [0054] *<br>* paragraph [0107] - paragraph [0210] *<br>* figures 1-8 * | 1-13 | TECHNICAL FIELDS SEARCHED (IPC)<br><br>A61B |
| A | EP 2 965 689 A1 (JVC KENWOOD CORP [JP])<br>13 January 2016 (2016-01-13)<br>* the whole document * | 1-13 | |
| A | US 2012/177266 A1 (TSUKIZAWA SOTARO [JP]<br>ET AL) 12 July 2012 (2012-07-12)<br>* abstract * | 1-13 | |

The present search report has been drawn up for all claims

| Place of search | Date of completion of the search | Examiner |
|---|---|---|
| Munich | 11 October 2017 | Tommaseo, Giovanni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 16 7719

This annex lists the patent family members relating to the patent documents cited in the above-mentioned European search report.
The members are as contained in the European Patent Office EDP file on
The European Patent Office is in no way liable for these particulars which are merely given for the purpose of information.

11-10-2017

| Patent document cited in search report | | Publication date | Patent family member(s) | | Publication date |
|---|---|---|---|---|---|
| JP H0735966 | A | 07-02-1995 | NONE | | |
| JP 2015144635 | A | 13-08-2015 | NONE | | |
| WO 2015146491 | A1 | 01-10-2015 | AU 2015235511 | A1 | 10-11-2016 |
| | | | CN 106163418 | A | 23-11-2016 |
| | | | EP 3123943 | A1 | 01-02-2017 |
| | | | SG 11201607901Y | A | 29-11-2016 |
| | | | US 2017007120 | A1 | 12-01-2017 |
| | | | WO 2015146491 | A1 | 01-10-2015 |
| US 2007279590 | A1 | 06-12-2007 | US 2007279590 | A1 | 06-12-2007 |
| | | | WO 2005063114 | A1 | 14-07-2005 |
| EP 2965689 | A1 | 13-01-2016 | EP 2965689 | A1 | 13-01-2016 |
| | | | JP 2014195641 | A | 16-10-2014 |
| | | | US 2015374223 | A1 | 31-12-2015 |
| | | | WO 2014136803 | A1 | 12-09-2014 |
| US 2012177266 | A1 | 12-07-2012 | CN 102510734 | A | 20-06-2012 |
| | | | EP 2596746 | A1 | 29-05-2013 |
| | | | JP 5529660 | B2 | 25-06-2014 |
| | | | JP 2012024154 | A | 09-02-2012 |
| | | | US 2012177266 | A1 | 12-07-2012 |
| | | | WO 2012011221 | A1 | 26-01-2012 |

EPO FORM P0459

For more details about this annex : see Official Journal of the European Patent Office, No. 12/82

**EP 3 251 584 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7035966 A

- JP 2000010723 A